# EUROPEAN PATENT SPECIFICATION

(11) **EP 3 746 485 B1**
(45) Date of publication and mention of the grant of the patent: **22.11.2023**
(21) Application number: 19746926.5
(22) Date of filing: 01.02.2019
(51) Int. Cl.: C07K 16/34, A61K 39/395, G01N 33/50, G01N 33/53, G01N 33/80

(54) **ANTIBODY SCREENS USING TRANSGENIC ANTIGEN(S)**
ANTIKÖRPER-SCREENING MIT TRANSGENEN ANTIGENEN
CRIBLAGE D'ANTICORPS UTILISANT UN(DES) ANTIGÈNE(S) TRANSGÉNIQUE(S)

(30) Priority: 02.02.2018 US 201862625945 P
(43) Date of publication of application: 09.12.2020
(73) Proprietor: Bloodworks, Seattle, WA 98104 (US)
(72) Inventor: ZIMRING, James Charles, Seattle, Washington 98104 (US); WU, Yanyun, Seattle, Washington 98104 (US)
(74) Representative: Hobson, David James
(86) International application number: PCT/US2019/016405
(87) International publication number: WO 2019/152878

(56) References cited:
- EP-A1- 3 062 109
- WO-A2-2017/053703
- US-A1- 2008 274 497
- WATERMAN HAYLEY ET AL: "Use of Monoclonal Antibodies to RBC Antigens As a Therapeutic to Allow Incompatible Transfusion", BLOOD, AMERICAN SOCIETY OF HEMATOLOGY, US, vol. 126, no. 23, 3 December 2015 (2015-12-03), page 1140, XP086640198, ISSN: 0006-4971, DOI: 10.1182/BLOOD.V126.23.1140.1140
- SOKOL, RJ et al.: "Investigation of Patients with Autoimmune Haemolytic Anaemia and Provision of Blood for Transfusion", Journal of Clinical Pathology, vol. 48, no. 7, July 1995 (1995-07), pages 602-610, XP055628398,
- KUIJPER, RW et al.: "Localization of the Platelet-Specific HPA-2 (KO) Alloantigens on the N-Terminal Globular Fragment of Platelet Glycoprotein Iba", Blood, vol. 79, no. 1, 1 January 1992 (1992-01-01), pages 283-288, XP055628401,
- BOCKAMP, E et al.: "Tetracycline-Controlled Transgenic Targeting from the SCL Locus Directs Conditional Expression to Erythrocytes, Megakaryocytes, Granulocytes, and C-kit-Expressing Lineage-Negative Hematopoietic Cells", Blood, vol. 108, no. 5, 9 May 2006 (2006-05-09), pages 1533-1541, XP055628402,
- HALL, AM et al.: "Deletion of the Dominant Autoantigen in NZB Mice with Autoimmune Hemolytic Anemia: Effects on Autoantibody and T-Helper Responses", Blood, vol. 110, no. 13, 4 September 2007 (2007-09-04), pages 4511-4517, XP055628404,
- CHAPANIAN, R et al.: "Enhancement of Biological Reactions on Cell Surfaces via Macromolecular Crowding", Nature Communications, vol. 5, no. 4683, 20 August 2014 (2014-08-20), pages 1-28, XP055628407,

## Description

### FIELD OF THE DISCLOSURE

The current disclosure provides methods that allow efficient detection of antibodies existing within a subject. The methods utilize transgenic genetically-modified cells that express a limited number of antigens as claimed. The methods find a particular use as a mechanism to screen and identify appropriate blood units for transfusion.

### BACKGROUND OF THE DISCLOSURE

There are many scenarios where it would be beneficial to quickly and accurately identify antibodies existing in a subject's blood. For example, 112.5 million pints (units) of donated blood are collected globally every year for blood transfusions. Blood transfusions are used to replace blood lost due to surgery or injury, or if an illness prevents the body from making blood or making some blood components. For example, patients with poor oxygen saturation, severe anemia, symptoms of cardiovascular disease, cancer, or clotting deficiencies may need blood transfusions.

The major components of blood include: red blood cells (RBCs), which carry oxygen and help remove waste products; white blood cells, which are part of the immune system and help the body fight infections; platelets, which help blood to clot properly during a bleeding episode; and plasma, the liquid component of blood that holds blood cells in suspension. Serum refers to a liquid that separates from blood when it coagulates; compared to plasma, serum lacks clotting proteins but may contain clotting metabolites that arise from the clotting process. Whole blood transfusions with all parts of the blood are done, but much more common are transfusions of components of the blood, with RBCs being the most commonly transfused.

Whole blood collected from volunteer donors for transfusion into recipients is typically separated into components: red blood cells, white blood cells, platelets, and plasma, using apheresis, centrifugation procedures, or other known methods. Each of these separated blood components may be stored individually for later use and are used to treat a multiplicity of specific conditions and disease states. For example, the red blood cell component is used to treat anemia, the concentrated platelet component is used to prevent or control bleeding, and plasma is used frequently as a source of clotting factors for the treatment of congenital or acquired clotting factor deficiencies.

In cell separation procedures, there is usually some small percentage of other types of cells which are carried over into a separated blood component. When contaminating cells are carried over into a separated blood component in a high enough percentage to cause some undesired effect, the contaminating cells are considered to be undesirable. White blood cells, which may transmit infections such as HIV and CMV also cause other transfusion- related complications such as transfusion-associated Graft vs. Host Disease (TA-GVHD), alloimmunization and microchimerism.

In some cases, a person can donate their own blood for transfusion, such as before a scheduled surgery, but most transfusions involve blood donated by others. Before a person receives a transfusion from a donor, compatibility testing between the donor and recipient blood must be performed. Giving a recipient donated blood that is compatible (e.g., matches the recipient's blood type) is crucial, as immune system cells within the recipient's or donor's blood can attack cells that are not a match leading to a transfusion reaction. Transfusion reactions can range in severity. Mild transfusion reactions are similar to allergic reactions and can include hives, itching, nausea, chest pain, and/or fever. Severe transfusion reactions, however, can be fatal.

Compatibility testing can involve: (i) typing the blood types of the donor and recipient; and (ii) screening the donor and recipient's blood to identify antibodies in the donor's blood that may attack the recipient's blood as foreign and/or antibodies in the recipient's blood that may attack the donor's blood as foreign.

Blood types are based on substances called antigens on the surface of a person's RBCs. Antigens are what antibodies bind to cause transfusion reactions. Antigens on the surface of RBCs (RBC antigens) can be carbohydrates (sugars) and/or proteins. The most important antigens in blood typing are called A, B, and Rh (Rhesus), leading to the ABO and Rh status blood types.

Regarding the ABO blood type, a person is A positive if the A antigen is found on the surface of their RBCs; B positive if the B antigen is found on the surface of their RBCs; AB positive if the A antigen and the B antigen are found on the surface of their RBCs; and type O if neither the A antigen nor the B antigen are found on the surface of their RBCs. Regarding the Rh blood type, each person is either Rh-positive or Rh-negative denoting the presence or absence of Rh on the surface of their RBCs (Rh can also be referred to as the D antigen).

As indicated previously, antibodies are protective proteins produced by a person's immune system to fight foreign substances that have invaded the body, such as bacteria, viruses, or in the case of blood transfusions, foreign blood antigens. Thus, a person's blood type is not only defined by what antigens are present on the surface of RBCs but also by antibodies present in the blood plasma or serum. For example, a person with type A positive blood can have antibodies against the type B antigen. Similarly, a person with type B positive blood can have antibodies against the type A antigen. A person with type O blood can have antibodies against both the type A and type B antigens, while people with type AB blood have neither antibodies against type A nor type B antigens. Antibodies against A antigen, B antigen, or both, are called "expected" antibodies because they occur in a person's blood lacking the corresponding antigen(s) on their RBCs.

In contrast to the ABO system, anti-Rh antibodies are normally not present in the blood of people with Rh-negative RBCs, unless the blood of these people have been exposed to Rh-positive RBCs, such as occurs in individuals receiving one or more previous blood transfusions or due to an Rh-negative mother's pregnancy with an Rh-positive fetus.

Based on the foregoing blood types, rules have been developed that govern which blood types can be donated to which recipients. People with type O negative blood are considered universal RBC donors because they do not have the A, B, or Rh antigens that could trigger attack by antibodies in the recipients. People with type AB positive blood are considered universal RBC recipients because they do not have antibodies that will attack the A, B, and/or Rh antigens.

Some units of donor blood do not fully match a recipient's, even though they have the same ABO and Rh types. This is because there are other RBC antigens besides A, B, and Rh that can cause transfusion reactions. In fact, there are more than 340 antigens separated into 33 or more blood group systems, such as the Duffy, Kell, Kidd, MNS, and P blood group systems. Normally, in all blood group systems other than ABO, a person does not produce an antibody to an RBC antigen if the corresponding antigen is missing from their RBCs. When antibodies that recognize non-self antigens are produced, they are called "unexpected" antibodies, "atypical" antibodies, or "alloantibodies". People who are likely to have alloantibodies include people who have had multiple blood transfusions because they have been exposed to foreign RBC antigens with each transfusion, and women who have been exposed to foreign RBC antigens from the fetus during pregnancy. An RBC "alloantigen" is capable of inducing production of an alloantibody by individuals who lack the alloantigen.

Alloimmunization describes an immune response provoked in a transfused recipient by a donor alloantigen. Alloantigens include blood group substances (A, B, or AB) on erythrocytes and histocompatibility antigens expressed on white cells and platelets.

Due to the added complexity noted above, after a patient's blood is typed according to the ABO/Rh systems, a test called an antibody screen is performed to assess whether the patient's blood contains antibodies to antigens of non-ABO blood group systems. The antibody screen identifies potential alloantibodies that could complicate administering donor blood to the recipient.

To perform an antibody screen, RBCs are collected from the blood of multiple donors to screen a potential recipient's blood for alloantibodies to multiple potential RBC antigens. If an alloantibody is detected, further analysis is required to identify which antigen the alloantibody is reacting against. This follow-up analysis is difficult because every human has a rich mixture of RBC antigens and other molecules associated with the RBC antigens and it is not always possible to isolate a single human RBC antigen in the absence of all other antigens. For example, some antigens are inherited in grouped patterns. As such, the identification of antibody specificity down to a particular antigen requires complex and sequential combinatorial analysis. Even with the complex analyses that are undertaken, in some cases it is not possible to identify with precision the single antigen targeted by a recipient's alloantibody. The complexity of the situation is further compounded when a recipient has multiple alloantibodies reacting against multiple potential RBC antigens.

Faster, more precise, and less labor-intensive methods to match donor blood units with recipients' blood remain a significant need in blood transfusion medicine. Waterman Hayley et al (2015), Blood, 126(23) describes the use of monoclonal antibodies to RBC antigens as a therapeutic to allow incompatible transfusion. EP3062109 A1 describes an antibody detection method and system. WO 2017/053703 A2 describes a composition and methods for assessing sensitivity and specificity of antibody detection reagents.

### SUMMARY OF THE DISCLOSURE

The invention is defined in the appended claims.

The current disclosure provides a significant advance in blood transfusion medicine by providing a method as claimed that allows precise identification of red blood cell (RBC) antigens against which a recipient has antibodies. The method as claimed removes the need to rely on complex sequential combinatorial analyses. In particular embodiments, the method as claimed achieves this advance by utilizing non-human mammalian RBC that are genetically modified to express a limited number of human RBC antigens. In particular embodiments, non-human mammalian RBC are genetically modified to express a single type of human RBC antigen. Library RBC cell lines can be created, wherein each cell line expresses a different human RBC antigen of a different cluster of RBC antigens. Recipient blood can be tested against each cell line, and if a reaction occurs, the antigen creating the reaction can be quickly and accurately identified. The method as claimed makes identifying an appropriate RBC sample for transfusion more precise, increasing patient safety. More generally, it will be understood that the methods described herein can be applied to a human, by expressing human RBC antigen(s) in transgenic RBCs of a non-human mammal (exemplified herein with mouse RBCs).

In particular embodiments, the non-human mammalian RBCs can be modified to express any antigen from any of the known human blood group systems. In particular embodiments, the transgenic non-human mammalian RBCs are transgenic mouse RBCs. In particular embodiments, the transgenic non-human mammalian RBCs express human blood group antigens selected from RhD, RhC, Rhe, RhE, Rhe, Fy^{a}, Fy^{b}, K, k, S, s, glycophorin A, glycophorin B, Jk^{a}, and/or Jk^{b}.

In some instances, human antibodies can react against non-relevant antigens on non-human mammalian RBCs. For example, human antibodies can react against carbohydrates found on the surface of mouse RBCs, and these reactions can detract from the precision and simplicity of the disclosed methods if not accounted for. To overcome these issues, a pre-screen is conducted wherein antibody(s) that bind the confounding non-human antigen(s) are bound and subsequently removed from the patient blood sample, before the primary testing screen. In particular embodiments, another approach to address this issue is to further modify the non-human mammalian RBCs so that they do not express the confounding antigen. In particular embodiments, yet another approach to address this issue is to provide a masking agent that "covers" the confounding antigen so that it is not recognized by antibodies within the patient blood sample.

The methods provided herein are applicable more broadly than in the context of blood transfusion.

Embodiments herein are methods of detecting an alloantibody in a human blood sample (such as a serum or plasma sample, or another blood fraction that includes antibodies but from which a substantial portion of the red blood cells have been removed), including: providing a transgenic red blood cell (RBC), from a non-human mammal, that expresses a human alloantigen on the surface of the transgenic RBC; providing a control RBC from a non-human mammal of the same species, that does not express the human alloantigen; contacting the control RBC with the human blood sample; removing the control RBCs to generate a pre-absorbed human blood sample; and contacting the transgenic RBC with the pre-absorbed human blood sample; and determining whether the human alloantigen is bound by an alloantibody in the pre-absorbed human blood sample, wherein such binding is indicative of presence of the alloantibody in the human blood sample.

In one embodiment, the method is a method for identifying one or more antibodies, and wherein determining that the antibody binds to the antigen identifies that antibody as specific for that antigen.

The invention provides a method comprising contacting a control RBC with a sample; removing (for instance, using centrifugation) the control RBC to generate a pre-absorbed sample; and contacting at least one transgenic RBC with the pre-absorbed sample; and determining whether an antibody binds to an antigen; wherein the at least one transgenic RBC, has been provided from, or derived from, a non-target vertebrate animal, that expresses the antigen from a target species other than the non-target vertebrate animal; wherein the sample is known or suspected to include the antibody that binds to the antigen and wherein the sample has been provided from the target species; wherein the control RBC has been provided from the non-target vertebrate animal of the same species that does not express the antigen; and wherein the target species is human and the non-target vertebrate animal is a non-human mammal. Optionally, where the biological sample to be contacted is a blood sample.

The invention provides a composition including: a transgenic red blood cell (RBC), from a non-target vertebrate animal, that expresses (for instance, on the surface of the RBC) an antigen from a target species other than the non-target vertebrate animal; an antibody bound to the antigen, wherein the antibody is from a sample from the target species that has been pre-cleared of unwanted antibodies using a non-transgenic RBC preparation from the non-target vertebrate animal; and wherein the target species is human and the non-target vertebrate animal is a non-human mammal (such as a mouse, a rabbit, a goat, or a rat).

Described herein is a composition including: a complex produced by a method including: providing at least one red blood cell (RBC), from a non-target vertebrate animal, that expresses an antigen from a target species other than the non-target vertebrate animal; providing a sample known or suspected to include an antibody (for instance, a plasma or serum, milk, saliva, etc.), which sample is from the target species; contacting the RBC with the sample to form the complex. The method may further include, prior to contacting the RBC with the sample to form the complex: providing a control RBC from the non-target vertebrate animal that does not express the antigen; contacting the control RBC with the sample; removing (for instance, involving centrifugation) the control RBCs to generate a pre-absorbed sample; and using the pre-absorbed sample in contacting the RBC with the sample to form the complex.

In one embodiment of the composition as claimed, the sample that is contacted with transgenic RBCs in various examples is selected from the group consisting of: plasma, serum, blood, milk, saliva, urine, tissue, tissue homogenates, or lysates.

In one embodiment of the composition as claimed, contacting may include mixing the (transgenic) RBC and the sample in a container selected from the group consisting of: a test tube, a microcentrifuge tube, a multiwell plate, and a microfluidic device.

### BRIEF DESCRIPTION OF THE SEVERAL VIEWS OF THE DRAWINGS

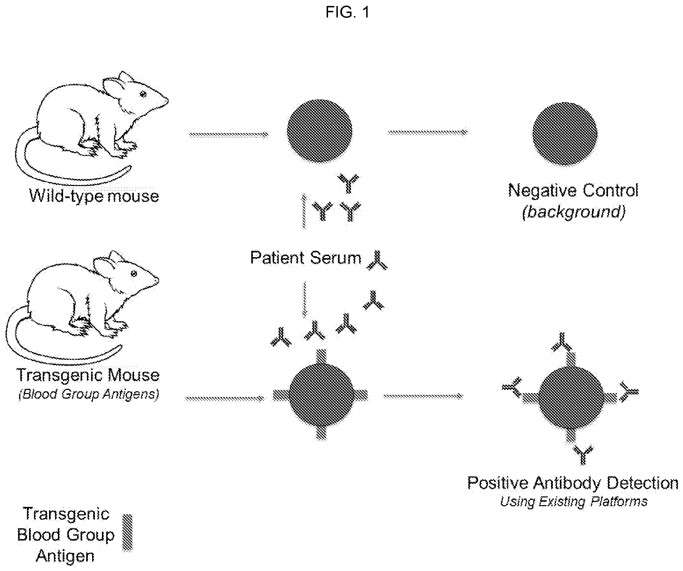

FIG. 1 is a diagram illustrating an embodiment of the herein-described isolated RBC antigen identification approach (isolated RBC antigen screen, IRAS). In the illustrated embodiment, transgenic mouse RBCs expressing at least one human antigen on their surfaces are incubated with human serum. RBCs from wild-type mice (target antigen negative, that is, not expressing a transgenic human RBC surface antigen) are used as a negative control for background antibodies. The described transgenic RBCs can be used as a detection reagent, for instance in any of the currently utilized (blood) antigen detection systems that use human RBCs, or can be adapted to new systems.
FIG. 2 is a Table (on two pages) of blood group systems derived from International Society of Blood Transfusion website at isbtweb.org.
FIG. 3 is a Table of blood group collections derived from International Society of Blood Transfusion website at isbtweb.org.
FIG. 4 is a Table of low incidence antigens (700 series) derived from International Society of Blood Transfusion website at isbtweb.org.
FIG. 5 is a Table of high incidence antigens (901 series) derived from International Society of Blood Transfusion website at isbtweb.org.
FIG. 6 is a Table (on four pages) showing identified alloantigens; this table was derived from the Table of Blood Group Antigens v.6_170205 (accessed Feb. 2, 2018), available online at isbtweb.org/fileadmin/user_upload/Working_parties/WP_on_Red_Cell_Immunogenetics_and/Ta ble_of blood_group antigens within_systems v6_170205.pdf.
FIG. 7 is a Table showing platelet antigens.
FIG. 8 illustrates flow cytometry analysis of RBCs isolated from Jk^{b} transgenic mice that express human Jk^{b} on their RBCs. Shown is staining for wild-type murine RBCs that express no Jk^{b} as a negative control (left peak, diamond), Jk^{b} transgenic murine RBCs (middle peak, triangle), and human RBCs that endogenously express Jk^{b} (right peak, circle).

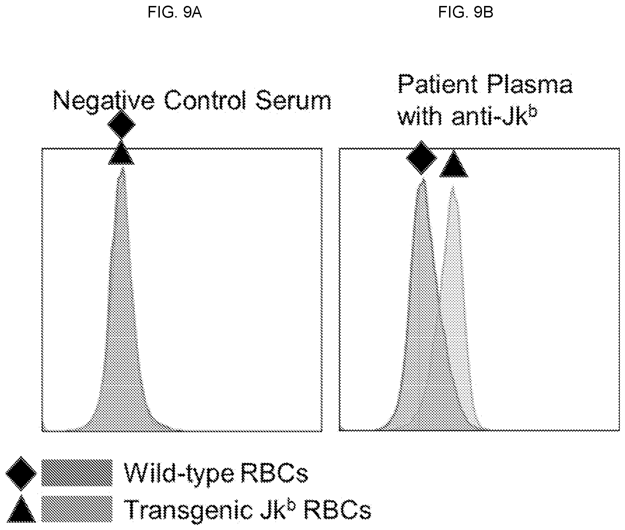

FIGs. 9A, 9B illustrate flow cytometry analysis using transgenic Jk^{b} RBCs to detect human alloantibodies in human serum. Patient plasma known to contain anti-Jk^{b} (FIG. 9B) and control patient plasma known to have no alloantibodies (FIG. 9A) were each absorbed with wild-type RBCs, followed by incubation with either wild-type antigen negative RBCs or transgenic Jk^{b} + RBCs. RBCs were then stained with fluorescently conjugated anti-human globulin and analyzed by flow cytometry. Histograms of wild-type and Jk^{b} transgenic RBCs were superimposable for the negative control plasma (FIG. 9A). Transgenic Jk^{b}+ RBCs (FIG. 9B, right peak, triangle) showed a positive shift compared to wild-type RBCs (FIG. 9B, left peak, diamond).

### DETAILED DESCRIPTION

There are a variety of scenarios in which it would be beneficial to screen a subject's blood for antibodies against particular antigens. For example, in some clinical scenarios, it would be beneficial to screen a subject's blood for antibodies that bind to particular infectious agents or autoimmune markers. Often, such screening is carried out with a blood fraction, for instance a blood fraction from which the majority of red blood cells has been removed; plasma and serum are particularly contemplated.

In the context of blood transfusions, rules have been developed that govern which blood types can be transfused into which recipients. People with type O negative blood are considered universal RBC donors because they do not have the A, B, or Rh antigens that could trigger attack by antibodies in the recipients. People with type AB positive blood are considered universal RBC recipients because they do not have antibodies that will attack the A, B, and/or Rh antigens.

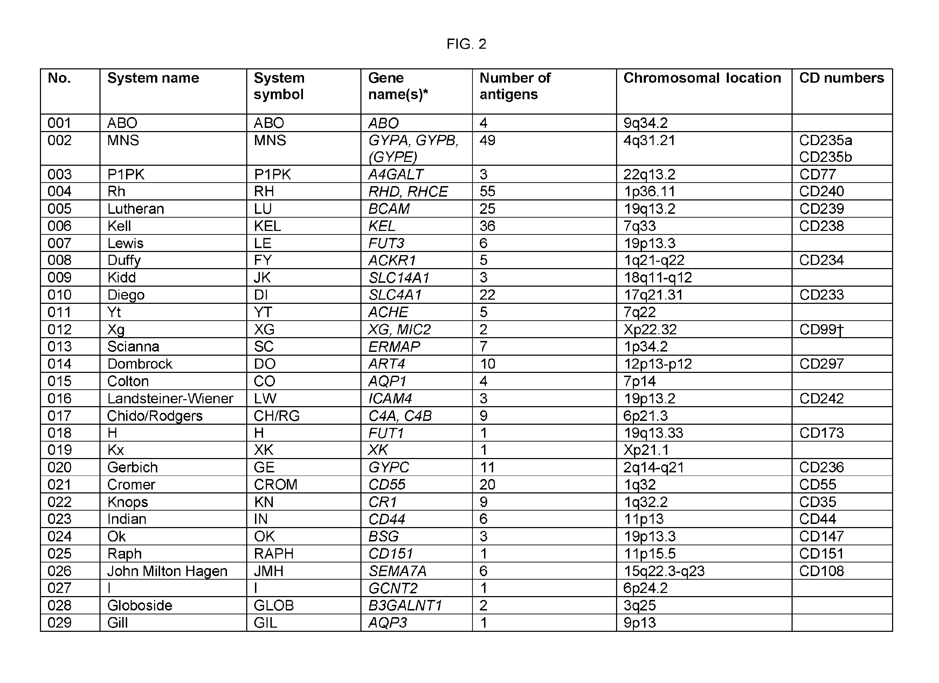

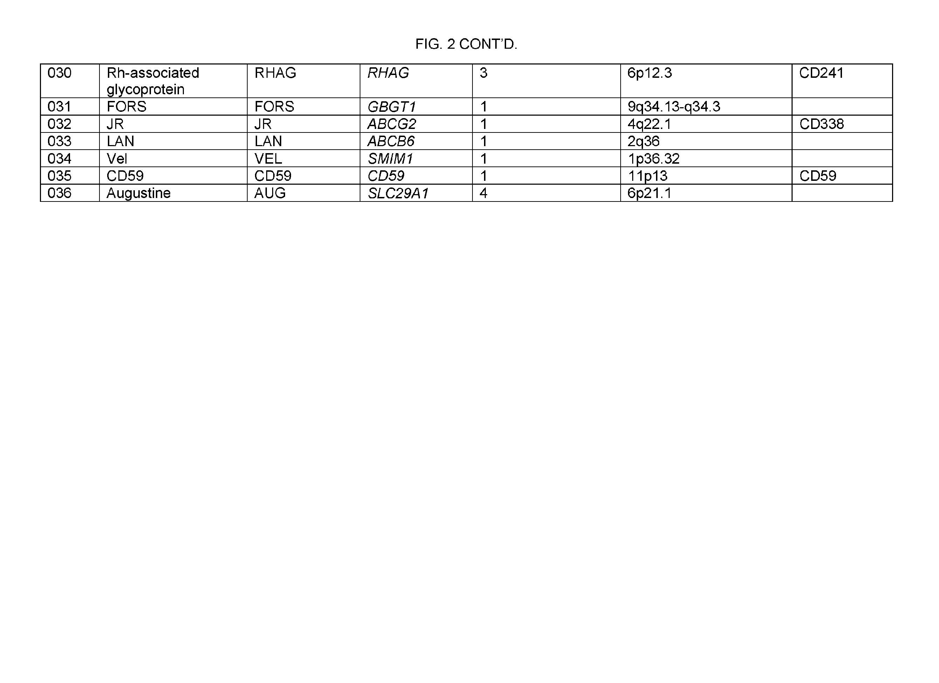

Some units of donor blood do not fully match a recipient's, even though they have the same ABO and Rh types. This is because there are other RBC antigens besides A, B, and that can cause transfusion reactions. In fact, there are over 340 antigens separated into 36 blood group systems as defined by the International Society of Blood Transfusion; see FIG. 2), such as the Duffy, Kell, Kidd, MNS, and P blood group systems. Normally, in all blood group systems other than ABO, a person does not produce an antibody to an RBC antigen if the corresponding antigen is missing from their RBCs. When antibodies that recognize non-self antigens are produced, they are called "unexpected" antibodies, "atypical" antibodies, or "alloantibodies". People who are likely to have alloantibodies include people who have had multiple blood transfusions because they have been exposed to foreign RBC antigens with each transfusion, and women who have been exposed to foreign RBC antigens from the fetus during pregnancy. An RBC "alloantigen" is capable of inducing production of an alloantibody by individuals who lack the alloantigen.

Due to the added complexity noted above, after a patient's blood is typed according to the ABO/Rh systems, human RBCs collected from the peripheral blood of living donors are used as a target to screen patient serum for antibodies to RBC antigens prior to transfusion. If an antibody is detected, subsequent identification of the target(s) recognized by the antibodies is likewise carried out with human RBCs from living donors. While a panel of 2-3 donor cells can be used for a general screen of the majority of clinically significant antibodies to known alloantigens, the follow-up identification of antibody specificity can be complicated. The difficulty lies in the fact that each human has the full complement of all the molecules that carry the different blood group antigens. Thus, while a single donor may have certain variations of such molecules (and as such have some antigens and not others), one does not have the ability to isolate a single human RBC antigen in the absence of all other antigens. As such, the identification of antibody specificity can be very challenging.

The current state-of-the-art for solving this problem is that one must harvest peripheral blood from many different living human donors, with different genetic patterns of blood group antigens, attempting to find multiple examples of reactivity (or lack thereof). Because certain antigens co-segregate genetically, it can be difficult, and at times impossible to find target RBCs that have one and not the other. Moreover, for other antigens that are either very rare or extremely common (in some cases essentially ubiquitous) it is likewise hard to find sufficient donors of the correct phenotype to properly characterize a patient's antibodies. This problem is greatly amplified in patients who have antibodies against multiple targets simultaneously, making the identification of the complex mixture very difficult (and at times impossible), without the ability to isolate one target at a time. This problem is only exacerbated within a blood group system, in which multiple antigens may be present on the same gene product, and as such no human may exist in whom one antigen is present without the other.

The current disclosure provides a significant advance in blood transfusion medicine by providing a method as claimed that allows for the precise identification of human red blood cell (RBC) or other antigens against which a recipient has antibodies. The method as claimed removes the need to rely on the described complex sequential combinatorial analyses.

In particular embodiments, the method as claimed achieves this advance by utilizing non-human mammalian RBC genetically modified to express a limited number of human RBC antigens. In particular embodiments, non-human mammalian RBC are genetically modified to express a single type of human RBC antigen or a different combination of human RBC antigens. Library RBC cell lines can be created wherein each cell line expresses a different human RBC antigen. Recipient blood can be tested against each cell line, and if a reaction occurs, the antigen creating the reaction can be quickly and precisely identified. The method as claimed makes identifying an appropriate RBC sample for transfusion into a recipient more accurate, safer for patients, faster, more consistent, large scale, and less prone to human error based on complex sequential combinatorial analyses.

It will also be understood that the method as claimed can be applied to any human target antigen (exemplified herein with RBC antigen(s)), by expressing the selected heterologous antigen(s) from a human in transgenic RBCs of a non-human mammal. By way of example, additional antigens (beyond RBC antigens) include HLA antigens (which methods
are useful in detecting high titer low avidity [HTLA] antibodies); antigens from cells other than RBCs (such as platelets, leukocytes, and so forth) that may be the source of alloantigens (which methods can be used to test for non-RBC alloantigens as well as to quantify or titer antibody levels); antigens of a microbial nature (e.g., viral or bacterial antigens) (which methods can be used for instance to test for serological response to infectious disease); autoantigens (such as DNA, Smith, etc.) (which methods can be used to test for autoantibodies, for instance in conditions such as autoimmune hemolytic anemia and other conditions in which the presence of both autoantibodies and alloantibodies can make blood typing particularly complex); human carbohydrate antigen(s) (for instance, through expressing one or more human glycosyltransferase(s) that permit expression on the non-human mammalian cells specific human carbohydrate antigen(s)); and others as described herein.

In particular embodiments, the non-human mammalian RBCs can be modified to express any antigen from any of the known human blood group systems. In particular embodiments, the transgenic non-human mammalian RBCs are transgenic mouse RBCs. In particular embodiments, the transgenic non-human mammalian RBCs express human blood group antigens selected from RhD, RhC, Rhe, RhE, Rhe, Fy^{a}, Fy^{b}, K, k, S, s, glycophorin A, glycophorin B, Jk^{a}, and Jk^{b}.

Transgenic mice expressing a single human blood group antigen on their RBCs have been generated for a number of purposes. Smith et al. (Transfusion 52:2620-2630, 2012) generated transgenic mice expressing human blood group antigens KEL1 and KEL2 and showed that the antigens were expressed in an RBC-specific and stable manner. Auffray et al. (Blood 97:2872-2878, 2001) used transgenic mice expressing the blood group antigen glycophorin A to serve as a model in which to explore the association of glycophorin A with another integral membrane protein, Band 3, in the mouse during erythroid membrane biogenesis. Halverson et al. (Transfusion 41:1393-1396, 2001) immunized transgenic mice expressing the Duffy Fy^{b} antigen with its antithetical counterpart, Fy^{a}, to generate monoclonal antibodies that recognizes the Fy^{a} antigen and not the Fy^{b} antigen. While these types of transgenic mice have been generated, only the current disclosure provides libraried application of the RBCs from such mice to the detection of human alloantibodies. The method as claimed revolutionizes RBC antibody screen and identification.

As depicted in FIG. 1, in particular embodiments, transgenic non-human mammals can be generated to express human antigens on the animal's RBCs. Mice, for instance, do not express (or express only very few) antigens recognized by human alloantibodies. As such, in particular embodiments, mouse RBC serve as a "blank canvas" upon which human RBC antigens can be expressed as targets to detect human alloantibodies. The RBCs from transgenic non-human mammals (individually expressing human blood antigens) can be incubated with serum or plasma from patients who are being tested for antibodies to human RBC antigens. Wild-type transgenic non-human mammals (lacking human antigens) can be utilized as a negative control to establish background staining. After incubation, human alloantibodies binding to the transgenic target non-human mammalian RBCs (but not control non-human mammalian RBCs) can be detected by a variety of existing platforms, including: 1) agglutination by tube assay; 2) gel card; 3) flow cytometry; 4) solid phase platforms; 5) spotted antigen arrays; and 6) silicon photonics, etc.

In particular embodiments, a selected transgenic non-human mammalian species may express certain human antigens that human antibodies will bind to, introducing confounding background noise into the method as claimed. In this scenario, approaches should be adopted that reduce this background signal. Exemplary approaches include removing such antibodies from the recipient sample before conducting the primary screen and/or removing the antigen from the cells of the transgenic non-human mammal.

There is provided herein in an embodiment a method of detecting an alloantibody in a human blood sample, including: providing a transgenic red blood cell (RBC), from a non-human mammal, that expresses a human alloantigen on the surface of the transgenic RBC; providing a control RBC from a non-human mammal of the same species, that does not express the human alloantigen; contacting the control RBC with the human blood sample; removing the control RBCs to generate a pre-absorbed human blood sample; and contacting the transgenic RBC with the pre-absorbed human blood sample; and determining whether the human alloantigen is bound by an alloantibody in the pre-absorbed human blood sample, wherein such binding is indicative of presence of the alloantibody in the human blood sample.

It is understood that the blood sample may be a blood fraction, such as a fraction of blood from which the majority of the red blood cells have been removed. Specifically contemplated blood fractions include blood serum and blood plasma. Thus, in an alternative embodiment, the method is a method of detecting an alloantibody in a human blood fraction that is serum or plasma, wherein the method involves providing a transgenic red blood cell (RBC), from a non-human mammal, that expresses a human alloantigen on the surface of the transgenic RBC; providing a control RBC from a non-human mammal of the same species, that does not express the human alloantigen; contacting the control RBC with the human serum or plasma sample; removing the control RBCs to generate a pre-absorbed human sample; contacting the transgenic RBC with the pre-absorbed human sample; and determining whether the human alloantigen is bound by an alloantibody in the pre-absorbed human sample, wherein such binding is indicative of presence of the alloantibody in the human serum or plasma sample.

In one embodiment, the method is a method for identifying one or more antibodies, and wherein determining that the antibody binds to the antigen identifies that antibody as specific for that antigen.

In embodiments of the method, the heterologous antigen is present on (that is, expressed onto) the cell surface of the transgenic non-human mammalian RBC.

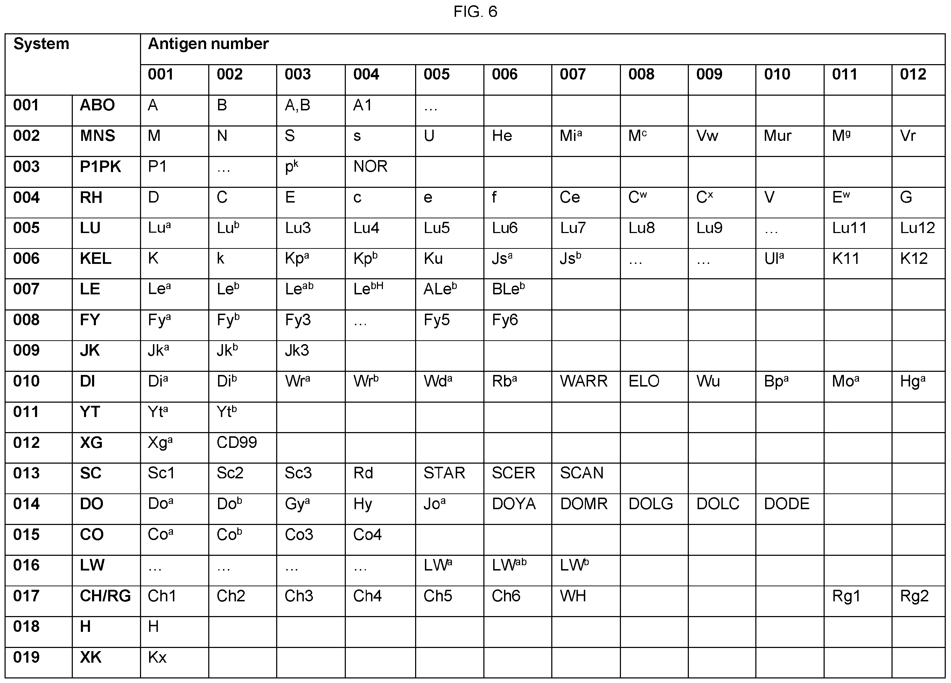

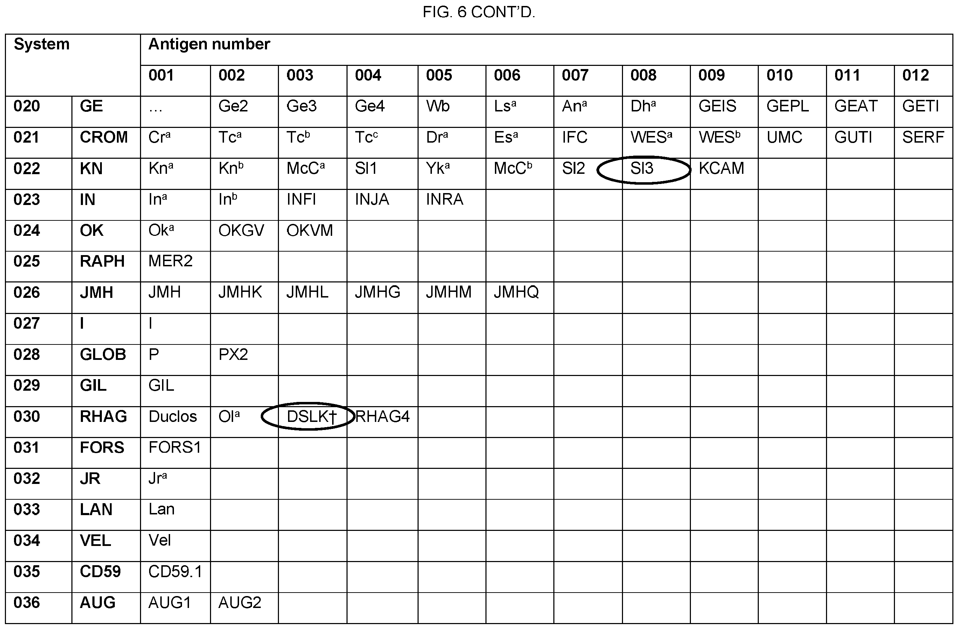

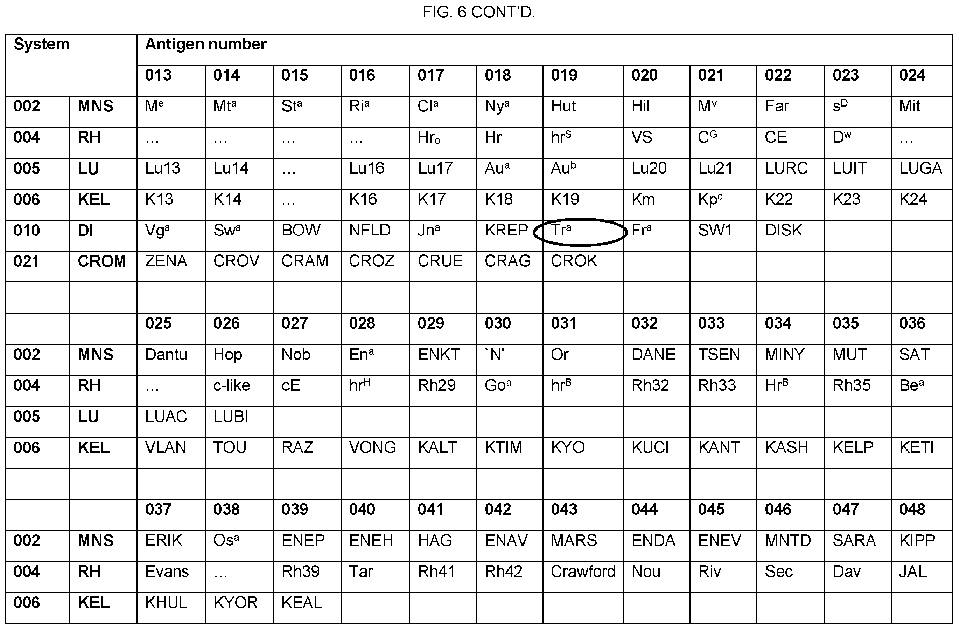

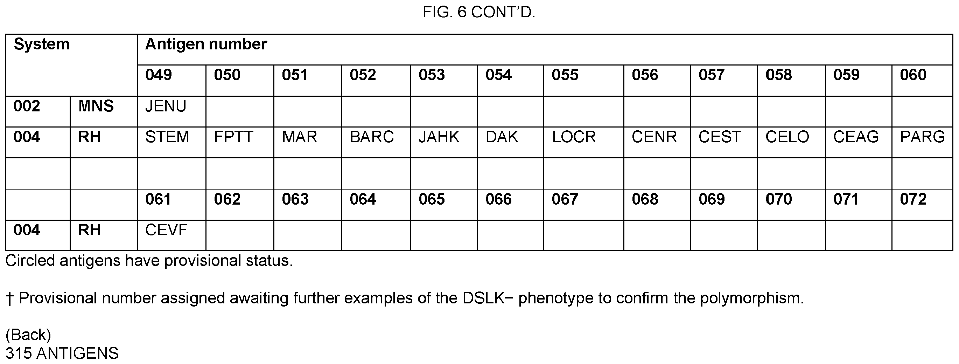

The method as claimed includes embodiments wherein the expressed, transgenic antigen includes an alloantigen. By way of example, the alloantigen is an alloantigen listed in FIG. 6. For instance, the alloantigen is in some cases selected from a blood group system consisting of: ABO, FY, KEL, JK, MNS, GLOB, and Rh systems. More specifically, in certain method embodiments the alloantigen is selected from the group consisting of: A, B, O, Fya, Fyb, KEL1, KEL2, KPb, KPa, Jsb, Jsa, Jka, Jkb, M, N , S, U, P, RhD, RhCE, Rhce, RhCe, RhcE, f, and G.

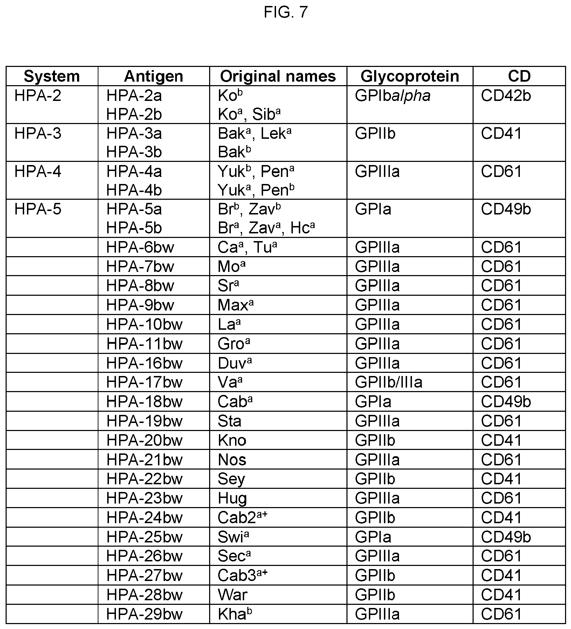

In additional embodiments, the alloantigen includes a platelet antigen. By way of example, the platelet antigen is a platelet antigen listed in in FIG. 7.

In yet more method embodiments, the antigen includes an autoantigen; or the antigen includes a microbial, fungal, viral, or bacterial antigen; or the antigen includes a fetal antigen.

Also contemplated are method embodiments, wherein the transgenic non-human mammalian RBC expresses two or more distinct antigens from a human.

In example method embodiments, the RBC (which expresses an antigen from a human) is isolated from a transgenic non-human mammal engineered to express the antigen. In additional embodiments, the (transgenic) RBC is derived from a stem cell isolated from a transgenic non-human mammal that is engineered to express the antigen. In yet additional embodiments, the (transgenic) RBC is derived from a stem cell isolated from a non-human mammal, wherein the stem cell is engineered to express the antigen. For instance, the transgenic non-human mammal in examples of these embodiments is further engineered to express one or more post-translational modification enzymes from a human. Optionally, the transgenic non-human mammal in various embodiments provided herein is further engineered to delete or inactivate one or more native proteins expressed in the RBC that are known or considered to be cross-reactive to one or more antibodies in the target sample.

In various embodiments of the methods provided herein, the sample is a biological sample selected from the group consisting of: plasma, serum, blood, milk, saliva, urine, tissue, tissue homogenate, and lysate.

In example method embodiments provided herein, contacting includes mixing the RBC and the sample in a container selected from the group consisting of: a test tube, a microcentrifuge tube, a multiwell plate, and a microfluidic device.

In example method embodiments provided herein, detecting includes an assay selected from the group consisting of: agglutination by tube assay, gel card, flow cytometry, solid phase platforms, spotted antigen arrays, and ELISA.

The invention provides a method comprising contacting a control RBC with a sample; removing (for instance, using centrifugation) the control RBC to generate a pre-absorbed sample; contacting at least one transgenic RBC with the pre-absorbed sample; and determining whether an antibody binds to an antigen; wherein the at least one transgenic RBC, has been provided from, or derived from, a non-target vertebrate animal, that expresses the antigen from a target species other than the non-target vertebrate animal; wherein the sample is known or suspected to include the antibody that binds to the antigen and wherein the sample has been provided from the target species; wherein the control RBC has been provided from the non-target vertebrate animal of the same species that does not express the antigen; and wherein the target species is human and the non-target vertebrate animal is a non-human mammal.

Yet additional embodiments of the provided method further include removing one or more native proteins expressed by the RBC that are known or considered to be cross-reactive to one or more antibodies in the sample prior to contacting the RBC with the sample. By way of example, removing one or more native proteins includes one or more of: treatment with periodic acid to remove carbohydrates; treatment with at least one glycosidase to remove carbohydrates; and treatment with at least one protease under conditions that remove cross-reactive antigens but do not remove the antigen from the target species.

The invention provides compositions that include: a transgenic red blood cell (RBC), from a non-target vertebrate animal, that expresses (for instance, on the surface of the RBC) an antigen from a target species other than the non-target vertebrate animal; and an antibody bound to the antigen, wherein the antibody is from a sample from the target species that has been pre-cleared of unwanted antibodies using a non-transgenic RBC preparation from the non-target vertebrate animal; and wherein the target species is human and the non-target vertebrate animal is a non-human mammal.

In example composition embodiments, the antigen expressed on the transgenic RBC includes an alloantigen. For instance, in specific examples the alloantigen is an alloantigen listed in FIG. 6. By way of example, the alloantigen is in certain embodiments selected from a blood group system consisting of: ABO, FY, KEL, JK, MNS, GLOB, and Rh systems. Specific examples include compositions in which the alloantigen is selected from the group consisting of: A, B, O, Fya, Fyb, KEL1, KEL2, KPb, KPa, Jsb, Jsa, Jka, Jkb, M, N , S, U, P, RhD, RhCE, Rhce, RhCe, RhcE, f, and G.

In additional composition embodiments, the alloantigen includes a platelet antigen such as a platelet antigen listed in FIG. 7.

In yet additional examples of compositions, the antigen includes an autoantigen; or includes a microbial, fungal, viral, or bacterial antigen; or includes a fetal antigen.

Also provided are composition embodiments, wherein the RBC expresses two or more distinct antigens from a human.

In additional composition embodiments, the RBC is isolated from a transgenic non-human mammal engineered to express the antigen.

In alternative composition embodiments, the RBC is derived from a stem cell isolated from a non-human mammal that is engineered to express the antigen.

The composition, in some embodiments, includes a RBC that is derived from a stem cell isolated from a non-human mammal, wherein the stem cell is engineered to express the antigen. For instance, in examples of this embodiment the transgenic non-human mammal is further engineered to express post-translational modification enzymes from the species other than the non-human mammal. Optionally, the transgenic non-human mammal is further engineered to delete or inactivate one or more native proteins expressed in the RBC that are known or considered to be cross-reactive to one or more antibodies found in a sample from the species other than the non-human mammal.

Also provided are composition embodiments, wherein the RBC has been treated to remove native proteins expressed by the RBC that are known or considered to be cross-reactive to one or more antibodies from the species other than the non-human mammal. Optionally, the treatment includes one or more of: treatment with periodic acid to remove carbohydrates; treatment with glycosidases to remove carbohydrates; and treatment with proteases under conditions that remove cross-reactive antigens but do not remove the antigen from the human.

Described herein is a composition including: a complex produced by a method including: providing at least one red blood cell (RBC), from a non-target vertebrate animal, that expresses an antigen from a target species other than the non-target vertebrate animal; providing a sample known or suspected to include an antibody, which sample is from the target species; contacting the RBC with the sample to form the complex.
The method may further include, prior to contacting the RBC with the sample to form the complex: providing a control RBC from the non-target vertebrate animal that does not express the antigen; contacting the control RBC with the sample; removing (for instance, involving centrifugation) the control RBCs to generate a pre-absorbed sample; and using the pre-absorbed sample in contacting the RBC with the sample to form the complex.

In any of the provided composition embodiments, the sample in various examples is selected from the group consisting of: plasma, serum, blood, milk, saliva, urine, tissue, tissue homogenates, or lysates.

In any of the provided composition embodiments, contacting may include mixing the (transgenic) RBC and the sample in a container selected from the group consisting of: a test tube, a microcentrifuge tube, a multiwell plate, and a microfluidic device.

Aspects of the current disclosure are now described in additional detail as follows: (I) RBC Antigens; (II) Other Transgenic Antigens; (III) Methods to Genetically Modify RBC to Express Heterologous Antigen(s); (IV) Methods to Reduce Confounding Background Signals; (V) Methods to Detect Presence of Antibodies; (VI) Samples for Analysis; and (VII) Uses and Alternatives of the Disclosure.

**(I) RBC Antigens.** In certain embodiments of the methods, and compositions as claimed, the transgenic antigen(s) expressed on the non-human mammalian RBC is a RBC antigen of a human. Representative examples of such RBC antigens are described below. Since RBC antigens are naturally expressed on the surface of red blood cells, these heterologous RBC antigens are expected to be expressed, at least in, part on the surface of the transgenic non-human mammalian RBCs.

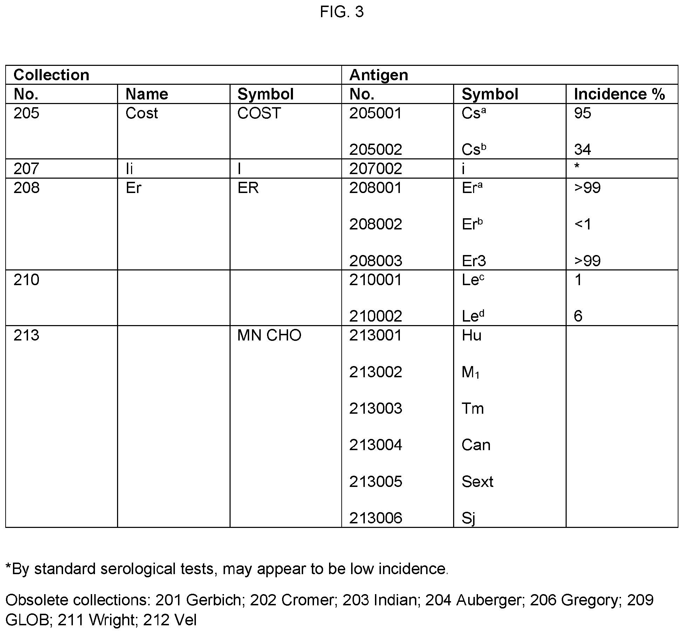

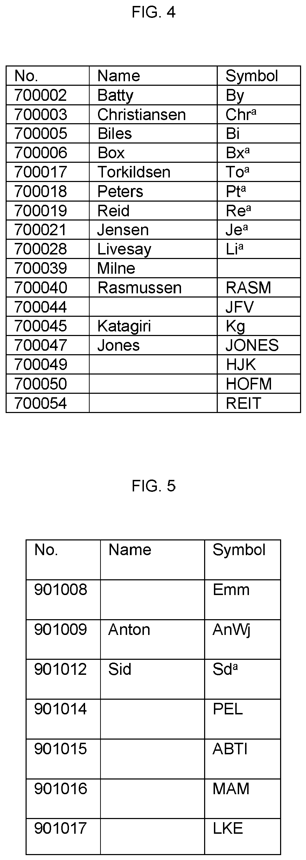

Thirty-four blood group systems were described in Patnaik et al. (Transfus Med Hemother 41:346-351, 2014). The International Society of Blood Transfusion (which defines 36 blood group systems) describes that authenticated RBC antigens fall into one of four classifications: 1) systems include one or more antigens controlled at a single gene locus, or by two or more very closely linked homologous genes with little or no observable recombination between them (FIG. 2, 36 blood group systems); 2) collections (200 series) include serologically, biochemically, or genetically related antigens, which do not fit the criteria required for system status (FIG. 3); 3) 700 series include low incidence antigens with an incidence of less than 1% in the population and cannot be included in a system or collection (FIG. 4); and 4) 901 series include high incidence antigens with an incidence of greater than 90% in the population and cannot be included in a system or collection (FIG. 5). The present disclosure encompasses creating transgenic non-human mammalian RBC antigen panels including any of the antigens in any of these 36 blood group systems, collections, 700 series low incidence antigens, or 901 series high incidence antigens. The genetic and molecular nature of many of the blood group antigens have been isolated, as well as the genetic variants that give rise to different antigens within a molecule (see FIGs. 6 and 7). Some common blood group systems are described below.

Kell (CD238) is a clinically important human blood group antigen system including 28 antigens. The Kell antigens are carried by a single pass type II RBC membrane glycoprotein having its N-terminus in the cytoplasm. The Kell glycoprotein is expressed in RBCs, hematopoietic tissue (bone marrow and fetal liver), and to a lesser extent in other tissues, including brain, lymphoid organs, heart and skeletal muscle. The K/k (KEL1/ KEL2) blood group antigen polymorphism is determined by a single nucleotide polymorphism (SNP) resulting in the presence of methionine (M) or threonine (T), respectively, at amino acid 193 of the extracellular C-terminal domain. The other most clinically significant antithetical (pair of) antigens Kp^{a}/Kp^{b} (KEL3/KEL4) and Js^{a}/Js^{b} (KEL6/KEL7) are also the result of SNPs resulting in single amino acid changes in the extracellular domain. Kell system antibodies are known to cause hemolytic transfusion reactions and hemolytic disease of the fetus and newborn (HDFN). Kell-related HDFN may be due to suppression of fetal erythropoiesis in addition to immune destruction of red blood cells as in most other cases of HDFN. Anti-K (KEL1) is the most commonly encountered immune red cell antibody outside the ABO and Rh systems, and other antigens of the Kell blood group system, e.g., k (KEL2), Kp^{a} (KEL3), Kp^{b} (KEL4), Js^{a} (KEL6) and Js^{b} (KEL7), are also capable of stimulating the production of hemolytic antibodies and causing HDFN.

The Duffy (Fy, CD234) blood group antigens are carried by a type III membrane glycoprotein, which is predicted to span the membrane seven times with a glycosylated extracellular N-terminus and a cytoplasmic C-terminus. It is expressed in red blood cells, vascular endothelial cells and a wide range of other tissues including kidney, lung, liver, spleen, brain, and colon. The Fy^{a}/Fy^{b} (FY1/FY2) blood group polymorphism is determined by a SNP resulting in the presence of glycine (G) or aspartic acid (D), respectively, at amino acid 42 in the N-terminal extracellular domain. Duffy blood group system antibodies can cause hemolytic transfusion reactions and HDFN.

The Lutheran (Lu, B-CAM, CD239) blood group antigens are carried by two single-pass type I (cytoplasmic C-terminus) membrane glycoproteins, which differ in the length of their cytoplasmic domains. The B-CAM glycoprotein has a shorter C-terminal cytoplasmic tail than the Lu glycoprotein. The Lu glycoprotein has five extracellular immunoglobulin-like domains and is a member of the immunoglobulin gene superfamily (IgSF) and is expressed in red blood cells and a wide range of other tissues. The Lu^{a}/Lu^{b} (LU1/LU2) blood group antigen polymorphism is determined by a SNP resulting in the presence of histidine (H) or arginine (R), respectively, at amino acid 77 of the first predicted N-terminal IgSF domain. Lutheran blood group system antibodies have been reported to be involved in mild delayed hemolytic transfusion reactions but are rarely involved in HDFN.

The Kidd (Jk) blood group antigens are glycoproteins present on the membrane of RBCs and act as urea transporters in RBCs and renal endothelial cells. Kidd (anti-Jk) antibodies are rare but can cause severe transfusion reactions, such as HDFN. Jk^{a} was the first antigen to be discovered in the Kidd blood group system. Two other antigens, Jk^{b} and Jk3, were subsequently found.

The M (MNS) blood group antigens include Glycophorin A and Glycophorin B. The blood group is under control of an autosomal locus on chromosome 4 and also under control of a pair of co-dominant alleles LM and LN. Anti-M and anti-N antibodies are usually IgM types and rarely associated with transfusion reactions.

The method as claimed is useful for expression of particular human RBC antigens of very high and/or very low frequency, for which it is exceedingly difficult to find living human donors that express or do not express the antigens. In particular embodiments, antigens listed in FIG. 4 can be expressed in a method as claimed.

The method as claimed is useful for expression of mutated or modified (non-naturally occurring) variants of blood group molecules specifically designed to eliminate some antigens and maintain others. In particular embodiments, a non-human mammalian transgenic cell expresses only one transgene antigen, for instance: only a Duffy Fy3 antigen; only a Duffy Fy^{a} antigen; only a Duffy Fy^{b} antigen; only an Rh G antigen; only an Rh C antigen; only an Rh D antigen; only a Kell K antigen; only a Kell k antigen; only a Kell Kp^{a} antigen; only a Kell Kp^{b} antigen; only a Kell Kp^{c} antigen; only a Kell Kp^{b} antigen; only a Kell Kp^{c} antigen; only a Kell Js^{a} antigen; or only a Kell Js^{b} antigen.

By way of example, such cells expressing such non-naturally occurring variants of blood group molecules can be used for simple titration, versus differential titration in the presence of antibodies against simple versus compound antigens. As such, in addition to identifying fine specificity of alloantibody, the relative amount of alloantibody to each particular antigen can be determined, as is useful to guide therapy and monitor immunity.

One of ordinary skill in the art can contemplate generating transgenic non-human mammalian cells (such as RBCs) to express any single antigen of those disclosed in FIGs. 2-7,
or any combination of 2, 3, 4, 5, 6, 7, 8, 9, 10, 11, 12, 13, 14, 15, 16, 17, 18, 19, 20, or more of those antigens. In particular embodiments, a combination of expressed antigens can be antigens from the same blood group systems. In particular embodiments, a combination of expressed antigens can be antigens from different blood group systems. In particular embodiments, a combination of expressed antigens can be all rare (or low incidence) antigens. In particular embodiments, a combination of expressed antigens can be all common (or high incidence) antigens. In particular embodiments, a combination of expressed antigens can be rare (or low incidence) and common (or high incidence) antigens. In particular embodiments, a combination of expressed antigens can be a combination found on particular donor cells.

Additional multi-antigen embodiments include systems in which the non-human mammalian cell expresses more than one antigen from a human, and there is provided a set of such transgenic non-human mammalian cells that together provide an entire set of the human antigens. A typical list of clinically significant antigens includes D, C, c, E, e, V, C^{w}, K, k, Kp^{a}, Kp^{b}, Js^{a}, Js^{b}, Fy^{a}, Fy^{b}, Jk^{a}, Jk^{b}, Le^{a}, Le^{b}, P1, M, N, S, s, Lu^{a}, Lu^{b}, and Xg^{a}. Thus, a set of transgenic non-human mammalian RBCs that together express all of these antigens is specifically provided, as are sets that include at least five of these antigens; at least 10 of these antigens; at least 12 of these antigens; at least 15 of these antigens; at least 18 of these antigens; at least 20 of these antigens; or more than 20 of these antigens.

By way of additional examples, in one embodiment there is provided a set of non-human mammalian cells that together express the entire or substantially the entire set of human RBC antigens (see, e.g., FIG. 2), or the entire or substantially the entire set of identified alloantigens shown in FIG. 6. For instance, a set of multi-antigen transgenic cells may express ten antigens each, such that the entire set of 340 human RBC antigens would be included in a cell-set having at least 34 different cells. In another embedment, the set of cells expresses collectively all of the platelet antibodies listed in FIG. 7. It will be clear that the size "set" of transgenic cells (that is, the number of cells needed in a set to represent the entire desired antigen set) is inversely proportional to the number of antigens that are expressed on each cell. Thus, a transgenic non-human mammalian cell set intended to represent 340 human RBC antigens may contain as few as one cell type (if all 340 antigens are expressed from the same cell) to as many as 340 total cell types (where each cell type expresses only and exactly one human RBC antigen). In sets of transgenic cells that are intended to provide expression of all (or substantially all) antigens in a desired human antigen set, it is contemplated that not all cells of the set will express the same number of antigens. Thus, there are contemplated sets in which some cells express only one transgenic antigen while another cell in the same set expresses more than one at the same time.

It is also contemplated that such multi-cell "sets" may include within the set more than one cell that expresses the same antigen. For instance, by expressing the same antigen as a transgene in multiple cells within a set can provide internal controls - such that screening a panel of the multi-transgenic cells permits higher confidence identification of positive signals when the expressed antigen is detected in the multiple cells in which it is expressed. Systems are also provided wherein the duplicative expression of antigens across a panel of transgenic non-human mammalian cells is used to reduce the overall number of cells needed to complete the "set" of antigens being tested. In such embodiments, it can be useful to employ computer algorithms to deconvolute signals where the co-expression of different antigens on transgenic cells in the set results in complex overlapping expression patterns.

**(II) Other Transgenic Antigens.** It will also be understood that the method as claimed can be applied to any human antigen (beyond the RBC antigen(s) discussed above), by expressing the selected heterologous antigen(s) from a human in transgenic RBCs of a non-human mammal.

By way of example, additional antigens (beyond RBC antigens) include HLA antigens (which methods are useful in detecting high titer low avidity [HTLA] antibodies); antigens from cells other than RBCs (such as platelets, leukocytes, and so forth) that may be the source of alloantigens (which methods can be used to test for non-RBC alloantigens, as well as to quantify or titer antibody levels); antigens of a microbial nature (e.g., fungal, viral, or bacterial antigens) (which methods can be used for instance to test for serological response to infectious disease or exposure to microorganisms); autoantigens (such as DNA, Smith, etc.) (which methods can be used to test for autoantibodies, for instance in conditions such as autoimmune hemolytic anemia and other conditions in which the presence of both autoantibodies and alloantibodies can make blood typing particularly complex); human
carbohydrate antigen(s) (for instance, through expressing one or more human glycosyltransferase(s) that permit expression on the non-human mammalian cells specific human
carbohydrate antigen(s)); and other antigens as described herein. Non-protein antigens and modified protein antigens are specifically contemplated, including glycoproteins and other post-translationally modified proteins.

Exclusive or preferential expression in RBCs of human antigen(s) is not required by the methods, and compositions as claimed. However, expression at the cell surface (and particularly, at the surface of RBCs) is beneficial. Thus, if the selected human antigen(s) to be expressed in non-human mammalian RBCs would not naturally be expressed at the cell surface (in the cell membrane), such antigens are beneficially expressed with a targeting sequence that directs cell surface (cell membrane) expression.

**(III) Methods to Genetically Modify RBCs to Express Heterologous (Transgenic) Antigen(s).** A transgenic non-target vertebrate animal (such as a non-human mammal) may functionally express one or more antigen(s) (such as RBC antigen(s), or other select antigen(s)) on the erythrocytes of the non-target vertebrate animal. Functional expression of an antigen includes expression of that antigen such that one or more biological activity of the antigen is maintained; such biological activity may be its ability to be recognized by an antibody. Similarly, functional expression of an RBC antigen includes expression of an RBC antigen such that biological activity of the RBC antigen is maintained, including expression at the surface (cell membrane) of the RBC. For example, Kell K antigen is "functionally expressed" on a cell when it is in a manner that results in Kell K antigen capable of binding to a natural ligand or to an antibody.

Constructs may be suitable for expression of a specified nucleic acid molecule (such as an antigen, such as a red blood cell surface antigen) in a host cell (which host cell is of a different species from the species the nucleic acid molecule is derived). Preferably, the constructs include one or more regulatory sequences (which may be selected on the basis of the host cells to be used for expression) that are operatively linked to the nucleic acid sequence(s) to be expressed. Within a recombinant expression vector, "operably linked" means that the nucleotide sequence of interest is linked to the regulatory sequence(s) in a manner which allows for expression of the nucleotide sequence in a host cell. Regulatory sequences include promoters, enhancers and other expression control elements (e.g., polyadenylation signals). Such regulatory sequences are described, for example, in Goeddel (1990) Gene Expression Technology: Methods in Enzymology 185, Academic Press, San Diego, CA. Promoters include regions of DNA involved in binding of RNA polymerase to initiate transcription.

Promoters that direct expression of antigen(s) on RBCs (preferentially or otherwise) can be used, include promoters that regulate expression of an RBC antigen endogenously (e.g., glycophorin A promoter to express the glycophorin; see, for instance, Vignal et al., Gene 95(2):289-293, 1990). However, expression exclusively in RBCs (or even preferentially in RBCs) is not essential, so long as the transgenic antigen is expressed in the RBC and preferably expressed at the surface of the RBC.

Expression of the target antigen(s) at the cell surface (and particularly, at the surface of RBCs) is beneficial. When the selected target antigen(s) would naturally be expressed at the cell surface (such as RBC surface antigens, or other antigenic proteins that would be expressed on a cell membrane when natively expressed), modification to direct cell membrane expression are generally not necessary. However, if the selected target species antigen(s) to be expressed in non-target species RBCs would not naturally be expressed at the cell surface (in the cell membrane), such antigens are beneficially expressed with a targeting sequence that directs cell surface (cell membrane) expression. Cell surface expression (that is, expression of cell membrane proteins) and methods for achieving it are well known in the art. For instance, protein localization signals such as plasma membrane localization sequences are known (Negi et al., Database 2015:bav003; doi:10.1093/database/bav003) (database available online at genome.unmc.edu/LocSigDB/). See, for instance, *"*Membrane Protein Expression in Mammalian Cells", Hizal, Ohsfeldt, Mai, & Betenbaugh, doi.org/10.1002/9783427634521.ch5, Chapter 5, Robinsin (ed)., 2011. Cell surface expression may include expression of an integral membrane protein, as well as peripheral membrane proteins, where at least the target antigenic portion of the protein is outside of the cell.

In particular embodiments, non-target transgenic non-human mammals have a heterologous or foreign gene incorporated into their genome. The term "transgenic animal" refers to a non-human mammal, *e.g.,* a swine, a monkey, a goat, or a rodent (e.g., a mouse, a rat, or a rabbit), in which one or more, and in some cases essentially all, of the cells of the animal include a transgene. The transgene is introduced into the cell, directly or indirectly by introduction into a precursor of the cell, *e.g.,* by microinjection, transfection or infection, *e.g.,* by infection with a recombinant virus. The term "genetic manipulation" includes the introduction of a recombinant DNA molecule. This molecule is preferably integrated within a chromosome to ensure that it will be passed on to offspring (of the cell, and/or of the animal), or it may be extrachromosomally replicating DNA.

In certain embodiments, the genome of a non-human mammal may contain a polynucleotide encoding at least one human antigen operably linked to a promoter such that the human antigen is functionally expressed on erythrocytes of the non-human mammal. In particular embodiments, a non-human mammal may have a genome containing a polynucleotide encoding at least one human RBC antigen operably linked to a promoter such that the human RBC antigen is functionally expressed on the erythrocytes of the animal.

The transgenic animal used in the methods of the disclosure is a non-human mammal. Suitable mammals for uses described herein include: rodents; ruminants; ungulates; domesticated mammals; and dairy animals. In particular embodiments, non-human animals include: rodents (such as rats and mice), rabbits, goats, sheep, camels, cows, pigs, horses, oxen, llamas, chickens, geese, and turkeys. In a specific embodiment, the non-human mammal is a mouse, a rabbit, or a goat.

Various methods of making transgenic animals are known in the art (see, *e.g*., Watson, et al. (1992) "The Introduction of Foreign Genes Into Mice," in Recombinant DNA, 2d Ed., W. H. Freeman & Co., New York, pp. 255-272; Gordon (1989) Intl. Rev. Cytol. 115:171-229; Jaenisch (1989) Science 240: 1468-1474; Rossant (1990) Neuron 2: 323-334). An exemplary protocol for the production of a transgenic pig can be found in White and Yannoutsos, Current Topics in Complement Research: 64th Forum in Immunology, pp. 88-94; US 5,523,226; US 5,573,933; WO 1993/025071; and WO 1995/004744. An exemplary protocol for the production of a transgenic rat can be found in Bader and Ganten (1996) Clinical and Experimental Pharmacology and Physiology, Supp. 3: S81-S87. An exemplary protocol for the production of a transgenic cow can be found in Transgenic Animal Technology, A Handbook, 1994, ed., Carl A. Pinkert, Academic Press, Inc. An exemplary protocol for the production of a transgenic sheep can be found in Transgenic Animal Technology, A Handbook, 1994, ed., Carl A. Pinkert, Academic Press, Inc.

**Injection into the Pronucleus.** Transgenic animals can be produced by introducing a nucleic acid construct into egg cells. The resulting egg cells are implanted into the uterus of a female for normal fetal development, and animals which develop and which carry the transgene are then backcrossed to create heterozygotes for the transgene. Embryonal target cells at various developmental stages are used to introduce transgenes.

Different methods are used depending on the stage of development of the embryonal target cell(s). Exemplary methods for introducing transgenes include microinjection of fertilized ovum or zygotes (Brinster et al., Proc. Natl. Acad. Sci. USA 82: 4438-4442, 1985), and viral integration (Jaenisch, Proc. Natl. Acad. Sci. USA 73: 1260-1264, 1976; Jahner et al., Proc. Natl. Acad. Sci. USA 82: 6927-6931, 1985; Van der Putten et al., Proc. Natl. Acad. Sci. (USA) 82: 6148-6152, 1985). Procedures for embryo manipulation and microinjection are described in, for example, Manipulating the Mouse Embryo (Cold Spring Harbor Laboratory Press, Cold Spring Harbor, NY., 1986). Similar methods are used for production of other transgenic animals, and particularly in larger species. Acceptable methods are known, see for instance Wheeler, M. B. (Transgenic Animals in Agriculture. Nature Education Knowledge 4(11):1, 2013).

In an exemplary embodiment, production of transgenic mice (for instance) employs the following steps. Male and female mice, from a defined inbred genetic background, are mated. The mated female mice are previously treated with pregnant mare serum, PMS, to induce follicular growth and human chorionic gonadotropin, hCG, to induce ovulation. Following mating, the female is sacrificed and the fertilized eggs are removed from her uterine tubes. At this time, the pronuclei have not yet fused and it is possible to visualize them using light microscopy. In an alternative protocol, embryos can be harvested at varying developmental stages, *e.g.* blastocysts can be harvested. Embryos are recovered in a Dulbecco's modified phosphate buffered saline (DPBS) and maintained in Dulbecco's modified essential medium (DMEM) supplemented with 10% fetal bovine serum.

Foreign DNA or the recombinant construct (*e.g.* an antigen expression construct) is then microinjected (100-1000 molecules per egg) into a pronucleus. Microinjection of an expression construct can be performed using standard micro manipulators attached to a microscope. For instance, embryos are typically held in 100 microliter drops of DPBS under oil while being microinjected. DNA solution is microinjected into the male pronucleus. Successful injection is monitored by swelling of the pronucleus. Shortly thereafter, fusion of the pronuclei (a female pronucleus and a male pronucleus) occurs and, in some cases, foreign DNA inserts into (usually) one chromosome of the fertilized egg or zygote. Recombinant ES cells, which are prepared as set forth below, can be injected into blastocysts using similar techniques.

**Embryonic Stem Cells.** In another method of making transgenic animals, recombinant DNA molecules can be introduced into mouse (or another non-target species) embryonic stem (ES) cells. Resulting recombinant ES cells are then microinjected into mouse blastocysts using techniques similar to those set forth in the previous subsection.

ES cells are obtained from pre-implantation embryos and cultured *in vitro* (Evans et al., Nature 292: 154-156, 1981); Bradley et al., Nature 309: 255-258, 1984; Gossler et al., Proc. Natl. Acad. Sci. (USA) 83:9065-9069, 1986; Robertson et al., Nature 322:445-448, 1986). Any ES cell line that is capable of integrating into and becoming part of the germ line of a developing embryo, so as to create germ line transmission of the targeting construct, is suitable for use herein. For example, a mouse strain that can be used for production of ES cells is the 129J strain. A preferred ES cell line is murine cell line D3 (American Type Culture Collection catalog no. CRL 1934). The ES cells can be cultured and prepared for DNA insertion using methods known in the art and described in Robertson, Teratocarcinomas and Embryonic Stem Cells: A Practical Approach, E. J. Robertson, ed. IRL Press, Washington, D.C., 1987, in Bradley et al., Current Topics in Devel. Biol., 20:357-371, 1986 and in Hogan et al., Manipulating the Mouse Embryo: A Laboratory Manual, Cold Spring Harbor Laboratory Press, Cold Spring Harbor, NY, 1986.

The expression construct can be introduced into the ES cells by methods known in the art, *e.g.,* those described in Sambrook et al., Molecular Cloning: A Laboratory Manual, 2nd Ed., ed., Cold Spring Harbor laboratory Press: 1989.

Suitable methods include electroporation, microinjection, and calcium phosphate treatment methods. The expression construct (*e.g.* heterologous antigen) to be introduced into the ES cell is preferably linear. Linearization can be accomplished by digesting the DNA with a suitable restriction endonuclease selected to cut only within the vector sequence and not within the gene (*e.g.* heterologous antigen gene, such as a red blood cell antigen from a target animal) or regulatory sequence (*e.g.* GATA-1 regulatory sequence).

After introduction of the expression construct, the ES cells are screened for the presence of the construct. The cells can be screened using a variety of methods, including methods of directly detecting the heterologous antigen(s) encoded by the construct using standard detection methods, including representative methods described herein. Where a marker gene is employed in the construct, the cells of the animal can be tested for the presence of the marker gene. For example, where the marker gene is an antibiotic resistance gene, the cells can be cultured in the presence of an otherwise lethal concentration of antibiotic (*e.g.,* G418 to select for neo). Those cells that survive have presumably integrated the transgene construct. If the marker gene is a gene that encodes an enzyme whose activity can be detected (*e.g.,* β-galactosidase), the enzyme substrate can be added to the cells under suitable conditions, and the enzymatic activity can be analyzed. Alternatively, or additionally, ES cell genomic DNA can be examined directly. For example, the DNA can be extracted from the ES cells using standard methods and the DNA can then be probed on a Southern blot with a probe or probes designed to hybridize specifically to the transgene. The genomic DNA can also be amplified by PCR with probes specifically designed to amplify DNA fragments of a particular size and sequence of the transgene such that, only those cells containing the targeting construct will generate DNA fragments of the proper size.

Implantation. The zygote harboring a recombinant nucleic acid molecule (*e.g.* a heterologous antigen, including control sequence(s) useful for expressing that antigen in red blood cells) is implanted into a pseudo-pregnant female mouse that was obtained by previous mating with a vasectomized male. In a general protocol, recipient females are anesthetized, paralumbar incisions are made to expose the oviducts, and the embryos are transformed into the ampullary region of the oviducts. The body wall is sutured and the skin closed with wound clips. The embryo develops for the full gestation period, and the surrogate mother delivers the potentially transgenic mice. Finally, the newborn mice are tested for the presence of the foreign or recombinant DNA. Of the eggs injected, on average 10% develop properly and produce mice. Of the mice born, on average one in four (25%) are transgenic for an overall efficiency of 2.5%. Once these mice are bred they transmit the foreign gene in a normal (Mendelian) fashion linked to a mouse chromosome.

**Screening for the Presence of the Transgenic Construct.** Transgenic animals can be identified after birth by standard protocols. DNA from tail tissue can be screened for the presence of the transgene construct, *e.g.,* using southern blots and/or PCR. Offspring that appear to be mosaics are then crossed to each other if they are believed to carry the transgene in order to generate homozygous animals. If it is unclear whether the offspring will have germ line transmission, they can be crossed with a parental or other strain and the offspring screened for heterozygosity. The heterozygotes are identified by southern blots and/or PCR amplification of the DNA. The heterozygotes can then be crossed with each other to generate homozygous transgenic offspring. Homozygotes may be identified by southern blotting of equivalent amounts of genomic DNA from mice that are the product of this cross, as well as mice that are known heterozygotes and wild type mice. Probes to screen the southern blots can be designed based on the sequence of a target organism antigen gene (such as a human or other target organism red blood cell antigen, or other types of antigens described herein), or the marker gene, or both.

Other means of identifying and characterizing the transgenic offspring are known in the art. For example, western blots can be used to assess the level of expression of the gene introduced in various tissues of these offspring by probing the western blot with an antibody against the protein encoded by the gene introduced (*e.g.,* a human or other target organism antigen protein), or an antibody against the marker gene product, where this gene is expressed.

*In situ* analysis, such as fixing the cells and labeling with an antibody, and/or FACS (fluorescence activated cell sorting) analysis of various cells, *e.g.* erythrocytes, from the offspring can be performed using suitable antibodies to look for the presence or absence of the transgene product. For example, to verify expression of human Jk^{b} on a non-human RBC/erythrocyte, flow cytometry can be performed using antibodies specific for human Jk^{b} (*e.g*. a commercially available monoclonal antibody such as: Seraclone^{®} Anti-Jk^{b} (JK2) from BioRad; Anti-Jk^{b} Gamma-clone^{®} from Immuncor; BIOSCOT^{®} anti-Jk^{b} (clone MS-8) from Millipore Sigma; and so forth) that are directly conjugated or used in conjunction with a secondary antibody that is fluorophore-conjugated and recognizes the antibody for Jk^{b}. In this analysis, human erythrocytes can be used as a positive control and normal (non-transgenic) mouse erythrocytes can be used as a negative control for the presence of Jk^{b}. Antibodies to most of the potential alloantigens are commercially available, for instance from Millipore, Immucor, BioRad, and/or Ortho Diagnostics. For antigen targets that do not have commercially available antibodies to confirm expression, patient sera with the desired specificity can be obtained from the network of International Reference Laboratories in bloodbanking.

**Animals Containing Multiple Antigen Transgenes or Additional Mutation(s).** Transgenic animals (such as mice) expressing one heterologous (target-organism derived) antigen (such as a human RBC antigen, or another type of antigen described herein) on their circulating erythrocytes as described herein can be crossed with another animal of the same species that a) harbors one or more additional transgene(s) (such as another transgenic antigen expressed on their RBC), or b) contain mutation(s) in other gene(s). Transgenic animals that are heterozygous or homozygous for each of the mutations can be generated and maintained using standard crossbreeding procedures. Examples of mice that can be bred with mice containing a target-organism antigen transgene include mouse strains which express human antigens and mice with targeted deletions of gene products that are themselves xenoantigens or that synthesize xenoantigens (*e.g.,* glycosyltransferases).

Also specifically contemplated are procedures to breed together different mice that each express a single human RBC alloantigen transgene, in order to generate transgenic RBCs that express multiple human alloantigens; such multi-antigen transgenic RBCs enable a broader screening cell (in addition to the single antigen expressing RBCs described herein, which useful for identifying an antibody once picked up by the screen).

Another specific example transgenic animal is a mouse is that expresses one or more heterologous antigens (such as RBC antigens) along with a knock-in transgene targeted to the ROSA26 locus (Friedrich & Soriano, Genes & Devel 5(9):1513-1523, 1991), in order to allow RBCs with higher and more uniform expression. See also Casola (Meth Mol. Biol., 667:145-163, 2010); Hohenstein et al. (PathoGenetics 1:3, 2008); and Kong et al. (PlosONE doi.org/10.1371/journal.pone.0107945, 2014).

The disclosure further pertains to cells derived from transgenic animals. Because certain modifications may occur in succeeding generations due to either mutation or environmental influences, such progeny may not, in fact, be identical to the parent cell, but are still included within the scope of the term as used herein.

**Non-Murine Transgenic Animals.** Though exemplified largely using mice as a representative transgenic system, use of non-murine non-target animals is expressly contemplated. For instance, the method as claimed is specifically contemplated with transgenic rabbits as the non-human mammal, which express on their RBCs one or more human antigens. Methods are known in the art for generating transgenic rabbits; see, for instance, Besenfelder, Aigner, Müller, & Brem (Generation and Application of Transgenic Rabbits. In: Cid-Arregui & García-Carrancá (eds) Microinjection and Transgenesis. Springer Lab Manual. Springer, Berlin, Heidelberg, 1998). Also contemplated are embodiments in which the non-human transgenic animal is a goat. Methods for generating transgenic goats are known in the art; see for instance, Baldassarre et al. (Reprod. Fertil. Dev. 16(4):465-470, 2004); Baldassarre et al. (Anim Reprod Sci 82-83:255-266, 2004). See also Wheeler (Nature Education Knowledge 4(11):1, 2013) and references cited therein for additional teachings on production of transgenic livestock.

Optionally, once a transgenic animal is generated that expresses the desired transgene(s), stem cells from the transgenic animal (such as a mouse) can be transplanted into another, different, and usually larger non-human mammal in order to provide a transgenic animal having a larger blood volume than the original transgenic mouse. For instance, it is contemplated that this type of stem cell transfer/bone marrow transplant can be used to produce transgenic rabbits, goats, and so forth that can produce larger volumes (compared to mice) of the desired transgenic RBCs. For a representative method of such bone marrow transplant, see Ildstad et al. (J. Experimental Medicine 174(2):467-470, 1991).

The method as claimed can include the isolation of pluripotent stem cells or hematopoietic stem cells from non-human mammals (e.g., mice, rabbits, goats, etc.) and culturing or expanding them under conditions that result in differentiation into mature RBCs (instead of using living non-human mammals as donors).

**(IV) Methods to Reduce Confounding Background Signals.** As described previously, in some instances, human antibodies can react against non-relevant antigens on non-human mammalian RBCs. For example, many humans have pre-existing (i.e. naturally occurring) IgG and/or IgM antibodies to mouse RBCs. These antibodies vary in levels from person to person, but can be present at a high enough frequency to confound the results of primary screens described herein. Particularly, human antibodies can react against carbohydrates found on the surface of mouse RBCs and these reactions can detract from the precision and simplicity of the disclosed systems and methods.

Pre-absorbing patient plasma or serum to wild-type non-human mammalian RBCs in order to remove antibodies that bind confounding non-human antigens. A pre-screen is conducted wherein antibodies that bind the confounding non-human antigens are bound and subsequently removed from the patient blood sample, before the primary testing screen. Particular embodiments reduce or eliminate background signal stemming from human antibodies recognizing mouse RBCs in an isolated RBC antigen screen (IRAS) by pre-absorbing patient plasma or serum to wild-type mouse RBCs to remove human antibodies recognizing mouse RBCs. In particular embodiments, a patient plasma or serum is incubated with wild-type mouse RBCs, the RBCs are removed from the plasma or serum by centrifugation, and the plasma or serum is then used in an antibody screen of the present disclosure.

Modifying non-human mammalian RBCs in order to remove the recognized antigen(s). In particular embodiments, non-human mammalian RBCs can be further modified so that they do not express the confounding antigen. For example, knock out mice can be generated that lack enzymes responsible for synthesis of one or more of the carbohydrates.

Treatment with periodic acid to destroy confounding non-human antigens that include carbohydrates. In particular embodiments, the treatment with periodic acid removes all carbohydrates.

Treatment with glycosidases to destroy confounding non-human antigens that include carbohydrates. In particular embodiments, mouse RBCs expressing a human RBC antigen can be treated with endo-β-galactosidase.

Treatment with proteases under conditions that destroy the non-human antigen recognized by human antibodies but do not destroy the RBC antigen expressed by non-human mammalian transgenic RBCs.

In particular embodiments, yet another approach to address this issue is to provide a masking agent that "covers" the confounding antigen so that it is not recognized by antibodies within the patient blood sample.

Particular embodiments reduce or eliminate background signal stemming from human antibodies recognizing mouse RBCs in an IRAS by treatment of transgenic mouse RBCs with periodic acid to destroy carbohydrates on the mouse RBCs. In particular embodiments, glucose and other saccharides are oxidatively cleaved in a Malaprade reaction by periodic acid due to an abundance of vicinal diol moieties on carbohydrates.

Also contemplated in embodiments of the antibody detection and identification methods provided herein is use of genetically engineered non-human mammals, in which one or more genes that lead to binding of naturally occurring human antibody(s) to non-human mammalian RBCs are deleted or disabled, thereby eliminating the need for absorption and/or enzymatic treatment of cells.

**(V) Methods to Detect Presence of Antibodies.** Diverse methods can be used to detect the presence of antibodies in patient plasma or serum that recognize the human antigen(s) (exemplified herein in certain embodiments by human RBC antigen(s)), including for confirmation of the systems provided herein. These methods include: (A) agglutination assay; (B) gel card; (C) flow cytometry; (D) solid phase platforms; (E) spotted antigen arrays; and (F) silicon photonics.

**Agglutination assay.** Agglutination is the clumping and sticking together of normally free cells or other small particles so as to form visible aggregates. This assay relies on the ability of antibodies to cross-link red blood cells by interacting with the antigens on the RBC surface. In particular embodiments, an agglutination assay can be performed in the following manner: test serum or plasma is serially diluted in tubes; a constant defined amount of transgenic non-human mammalian RBCs expressing a single human blood antigen is added to each series of tubes. The tubes are incubated at 37°C, and the assay can be read at 50% agglutination. Agglutination can be expressed as titer, or the inverse of the last serial dilution (lowest antibody concentration) that gives a positive result in an agglutination assay; *e.g.:* 1/1000. In particular embodiments, these assays can be performed in tubes, plates (*e.g.,* micro titer plates), or slides. In particular embodiments, serial dilutions of the sera or plasma being tested can be performed. Hemagglutination is agglutination of RBCs.

**Gel card.** The test combines the principles of hemagglutination and gel filtration for detection of blood group antigen-antibody reactions. The process can use special microtubes filled with a mixture of gel, buffer, and reagent. In particular embodiments, the test uses a neutral gel containing no reagents, and reagents are added to the top of the gel. In particular embodiments, a mixture of RBCs and serum is centrifuged through the gel under precise conditions. In negative reactions, non-agglutinated RBCs pass through the gel and pellet in the bottom of the tube; whereas, in positive reactions, agglutinates are dispersed throughout the length of the gel, depending upon their size. The reaction may be read for hours afterwards. The position of agglutinates in the gel determines the intensity of the reaction. The test is easy to perform, sensitive, and reproducible. Lapierre et al., Transfusion 30(2): 109-113, 1990.

**Flow cytometry.** Flow cytometry is a technique for analyzing and sorting cells (or other small particles) suspended in a fluid stream. This technique allows simultaneous analysis of the physical and/or chemical characteristics of single cells flowing through an optical, electronic, or magnetic detection apparatus. The flow cytometer includes of a flow cell which carries the cells in a fluid stream in single file through a light source with excites the fluorescently labeled detection marker(s) (for example, antibody reagents) and measures the fluorescent character of the cell. The fluid stream is then ejected through a nozzle and a charging ring, under pressure, which breaks the fluid into droplets. The flow cell device and fluid stream is calibrated such that there is a relatively large distance between individual cells or bound groups of cells, resulting in a low probability that any droplet contains more than a single cell or bound group of cells. The charging ring charges the droplets based on the fluorescence characteristic of the cell which is contained therein. The charged droplets are then deflected by an electrostatically-charged deflection system which diverts the droplets into various containers based upon their charge (related to the fluorescence intensity of the cell). A FACS system (e.g. the FACSARIA^{™} flow cytometer (BD Biosciences) and FLOWJO^{™} Version 7.6.4 (TreeStar)) can detect and record the number of total cells as well as the number of cells which display one or more fluorescent characteristics, e.g. the total number of cells bound by one or more antibody recognizing an RBC antigen. In the context of detection of blood group antigen-antibody reactions, transgenic non-human mammalian RBCs can be incubated with a recipient's plasma or serum. This can be followed by a second incubation with a fluorescently labeled anti-human immunoglobulin (lg) antibody. A blood group antigen-antibody interaction can be scored based on a comparison of the staining intensity produced by a recipient's plasma or serum with a normal control plasma or serum (i.e., a control known to not have any antibodies that react with transgenic non-human mammalian RBCs expressing a given blood group antigen).

**Solid phase platforms.** In solid phase testing, human RBC antigens expressed by non-human mammalian RBCs are bound to microplate wells, then patient plasma or serum is added, with incubation at 37°C. If antibodies against an RBC antigen(s) is present, the antibodies bind to antigen all over the bottom of the well. Unbound antibodies can be washed away. Then indicator RBCs coated with anti-immunoglobulin are added, which bind to the previously attached antibodies on the bottom of the well. A negative signal can exhibit as a solid "button" in the bottom of the well, indicating that there are no attached plasma or serum antibodies with which the anti-immunoglobulin coated indicator cells could bind. A strong positive signal (indicated as 4+) can exhibit as a diffuse "carpet" of indicator RBCs spread all across the bottom of the well, indicating that the plasma or serum antibodies are attached to the well-bound RBC antigens.

**Spotted antigen arrays.** Any antigen array format can be used, including planar arrays and bead-based arrays. Planar antigen arrays are generated by immobilization of a large number of different antigens in microspots, generally at a spatial density of up to 2000 per cm² or more. The antigens are immobilized on the solid support by means of either contact printing devices using, for example, pins that touch the surface of the solid support, or noncontact printing devices that involve jetting systems forming and propelling droplets onto the solid surface. Only minute amounts of capture reagent volume are spotted, usually in the range of 50-500 picoliters, resulting in spot sizes of 100-300 µm depending on the utilized solid support properties. Immobilization of the antigens may be physical, covalent or affinity-based. Bead-based arrays rely on immobilization of antigens on distinguishable microsphere sets as solid supports and detection of the captured antibodies on each microsphere set by means of a flow cytometric readout system. Microspheres utilized as solid supports carry functional groups, such as carboxyl or thiol groups, facilitating the immobilization of proteins.

**Silicon photonics.** Photonic devices can be used for detecting an analyte, such as a cell, antigen, or antibody, in a biological solution (*e.g.,* whole blood). Blood type can be determined through photonic sensing, for instance using a combination of direct detection of blood cells and serology. For representative methods and devices, see for instance WO 2013/013220 ("Photonic Blood Typing"); Kirk et al. (Blood 124:1565, 2014); Li et al. (Sensors & Activators B: Chemical 262:411-417, 2018).

### (VI) Representative Samples for Analysis

The compositions, and methods as claimed are useful for detecting and/or identifying one or more antibodies in a sample, such as biological sample. Such biological samples include blood, blood fractions from which red blood cells have been substantially removed (such as plasma and serum), milk, saliva, urine, tissue, tissue homogenates, and lysates. Such samples can be recently obtained or stored for a period of time (for instance, hours, days, weeks) before the analysis is carried out. Beneficially, stored samples are stored in an art-recognized manner that substantially preserves the presence and conformation of any antibodies presented therein. For instance, with blood and blood fraction samples, convention blood banking and blood component storage procedures are usually appropriate.

In certain embodiments, it is beneficial to prepare the sample before contacting it with the transgenic non-human mammalian RBCs in the analysis. By way of example, sample preparation may involve removing one or more components from the sample that might interfere with subsequent detection step(s). This is illustrated by removal of some or all (or substantially all) of the red blood cells from a blood sample before its analysis, as the RBCs from the test sample would undermine detection based on antibody binding to the transgenic non-human mammalian RBCs expressing the one or more human antigen(s). Substantial removal in this context means removal of at least 90% of the red blood cells originally present in the test sample; or removal of at least 95%, at least 97%, at least 98%, or more than 98% of the red blood cells originally present in the test sample. Another example of sample preparation involves removing cells or cell debris from a tissue sample, such as a tissue homogenate or tissue lysate. Methods for removing cells from such samples (including removing all or substantially all RBCs from a blood sample) are well known in the art.

Other sample preparation that is useful includes pre-clearing the test sample (such as a sample from a human subject or a patient) of unwanted antibodies using a non-transgenic non-human mammalian RBC preparation from the same non-human mammalian species as the transgenic RBCs. Such pre-clearing is described herein; see for instance Examples 1 and 2.

### (VII) Uses and Alternatives of the Disclosure

The transgenic RBCs described herein, which express one or more heterologous antigen(s), are used in various embodiments to detect the presence (or not) of antibody(s) that bind to that antigen in a biological sample. Where the transgenic antigen is known (as it is, since the RBC or the animal from which it was derived was intentional engineered to express that antigen), detecting binding of an antibody also provides identification of the antibody detected. That is, the binding (complexation) serves as identification of the antibody.

In certain embodiments, characterization of a sample (such as a blood sample) as containing a certain antibody using a method provided herein allows categorization of the sample so as to guide what therapy is appropriate for the patient from whom the sample was obtained. For instance, the methods and transgenic RBCs (and sets and collections of transgenic RBCs) described herein can be used to identify alloantibodies in patients, so that the appropriate unit of blood can be matched to a recipient. Once a sample from a subject/patient is characterized as containing (or not containing) an antibody, the presence of said antibody (or antibodies) is designated on the patient's medical record and used to guide which units of blood they receive based upon the presence or absence of donor antigens recognized by the recipient antibody(s) characterized.

Additional uses are contemplated, for the engineered non-human mammalian RBCs described herein. The following are a number of such contemplated uses.

**RBC Cell Membranes.** The non-human mammalian RBCs described herein, which express on their surface one or more heterologous antigens, can be used to prepare red blood cell membranes. For instance, non-human mammalian RBCs that express one or more human RBC or other antigen(s) can be converted to RBC membranes that can be used for antigen testing included in assays known in the art, including solid phase assays such as those discussed herein. Methods are known for isolating RBC membranes (ghosts); see, for instance, Hegedus et al. (Database 2015: 1/1/15 doi.org/10.1093/database/bav056) and references cited therein.

**High-Level Antigen Expression.** In particular embodiments, there are provided transgenic non-human mammalian RBCs that express high level target antigen(s) on their surface. Such high level expression can be accomplished, for instance, through use of over-expression constructs such as those integrated into a high-level expression site in the genome of the expression organism, or high level promoter or other control sequence(s). Specific expression of human blood group antigens at higher density than found on human RBCs can be used to increase sensitivity of detection assays. As a general rule, the higher the antigen density, the more sensitive the test. For certain human alloantigens, their natural expression is low, and as such, using human RBCs as targets can be relatively insensitive. By increasing expression, sensitivity can be increased by a method not available using naturally occurring human specimens.

**Elution of Antibodies.** In many cases, when a positive test results from patient antibodies binding to test RBCs, one elutes the antibodies off to allow further characterization. Thus, elution of antibodies from the non-human mammalian RBCs in order to further characterize them will be an important capability of the systems described herein. This is novel, as the elution will isolate a single specificity, in those embodiments where only a single antigen is expressed (such as a single alloantigen). This type specificity can never be guaranteed using natural (human) RBCs. Even in those embodiments in which more than one antigen is expressed transgenically on the engineered RBCs, the eluted antibodies would still be at most specific for only those few defined transgenic antigens that were so expressed. It is therefore expected that elution of antibodies from complexes formed between one or more antibodies from a sample from a human and a transgenic non-human mammal-derived RBCs (or the heterologous antigen(s) expressed thereon) will be useful in single antigen as well as multiple-antigen RBC embodiments.

**Detection of Non-IgG Isotypes.** Routine testing of human alloantibodies only detects the general presence of IgG, and likewise many of the exemplary discussions provided herein are related to detection of IgG(s). However, in some circumstances, determining non-IgG isotypes, or specific IgG subtype, can be very useful. The current technology can be adapted to detect these other antibody types, through the use of anti-human globulin specific for IgA, IgM or IgG subtype (in addition to standard anti-human globulin specific for IgG).

**Monitoring of Maternal Alloimmunization to Fetal Antigens.** A pregnant woman can become alloimmunized to fetal alloantigens, leading to hemolytic disease of the fetus and new born (in the case of RBCs) or neonatal alloimmune thrombocytopenia (in the case of platelets). The current technology can be used to monitor, detect, and characterize maternal alloantibodies to fetal antigens.

Similarly, when a lactating woman is alloimmunized to antigens, the antibodies are present in her milk. When an infant consumes the milk through nursing, the antibodies can enter the infant's blood stream and cause hemolysis in the infant (if the infant expresses the recognized antigen on RBCs). This problem extends not only to woman nursing babies, but also applies to "milk banks", where mother's milk is collected, stored, and then provided to infants in need thereof. Thus, the technology described herein can be used for testing of human milk for alloantibodies to RBCs. The current technology can be used to monitor, detect, and characterize maternal alloantibodies to fetal antigens in milk, enabling identification of milk that might be harmful to infants due to the presence of such antibodies. When identified as potentially harmful, the milk can be removed from (or prevented from being introduced into) a milk bank, or otherwise removed from possible consumption.

**Isolation of Target Cells.** The transgenic RBCs described herein, which express one or more heterologous antigen(s) from a human, can also be used to isolate cells from the human that bind to the antigens expressed on the RBCs. For instance, non-human mammalian (for instance, murine) RBCs that express one or more human RBC antigens can be used to isolate specific human cells that bind to those antigen(s), including T cells.

The Examples below are included to demonstrate particular embodiments of the disclosure. Those of ordinary skill in the art should recognize in light of the present disclosure that many changes can be made to the specific embodiments disclosed herein and still obtain a like or similar result.

### Example 1: Isolated Red Blood Cell (RBC) Antigen Screen

This example describes a representative embodiment in which RBCs from a transgenic mammal (exemplified as mice), each expressing a single alloantigen from a target organism (exemplified as human), are generated then used for screening blood from the target organism for the presence of antibodies to that alloantigen.

The genetic and molecular nature of most of the blood group antigens have been isolated, as well as the genetic variants of a given gene coding for a given RBC antigen that give rise to different versions of the RBC antigen. Using cDNA sequences, a panel of genetically engineered mice was generated, in which each separate line of mice expresses a single human blood group antigen on their RBCs. These RBCs can be harvested from the mice and used in place of the current human RBC panels for antibody screening and identification. This expression has been confirmed using antibodies specific to the human RBC antigens that have been engineered. In this way, transgenic murine RBCs have been isolated that express a single human RBC antigen at a time, which presents a solution to the problem of being able to identify and characterize antibodies from an antibody screen as described above. Although mice do have orthologues of many human genes of RBCs, mice do not naturally express most of the human blood group antigens. Moreover, many of the existing diagnostic platforms require the targets to specifically be RBCs or RBC membranes. The antigens of interest are expressed on RBCs. As such, isolated RBC antigen screen (IRAS) can be plugged directly into existing diagnostic platforms without alteration.

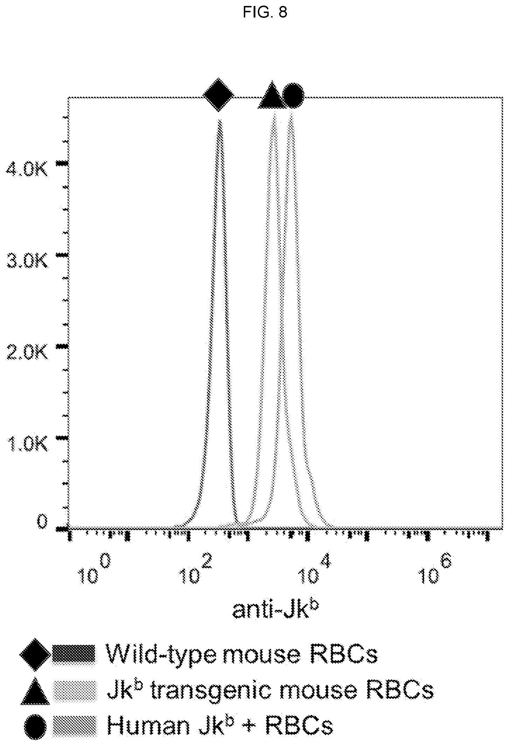

A novel transgenic mouse was created that expresses Jk^{b} on its RBCs (FIG. 8). RBCs from wild-type mice are negative for Jk^{b}, and constitute background staining. Jk^{b} transgenic mouse RBCs stain strongly positive with anti-Jk^{b}, albeit slightly weaker than human Jk^{b}+ RBCs (of note, additional mice could be created that express a higher level of Jk^{b}).

Identification of mouse RBC antigen recognized by human antibodies and validation of transgenic RBCs expressing K, k, or Jk^{b} alloantigens. Human plasma was incubated with wild-type mouse RBCs, and bound antibodies were eluted and tested for carbohydrate binding with a glycan array containing 600 different carbohydrates of known chemistry.

Wild-type mouse RBCs typed negative for RhD, RhC, Rhc, RhE, Rhe, Fy^{a}, Fy^{b}, K, k, S, s, or Jk^{b} antigens but were reactive with anti-Jk^{a}. "Naturally occurring" alloantibodies to mouse RBCs, both IgM and IgG, were detected in multiple samples of human plasma. Eluates from mouse wild-type RBCs incubated with human plasma were observed to have a strong IgG signal against only a single carbohydrate of the structure GalNAca1-3(Fuca1-2)Galb1-4GlcNAcb1-3Galb1-4GlcNAcb1-3Galb1-4GlcNAcb-; no reactivity was seen with other carbohydrates of a similar structure (*e.g.,* other poly-N-acetyl-lactosamines).

**Validation of IRAS.** Two human patient samples were analyzed. Sample 1 was negative control plasma in which there were no detectable antibodies against human RBC antigens. Sample 2 was from a patient who had an anti-Jk^{b} antibody detected by a clinical lab. Both plasma were absorbed with wild-type mouse RBCs to remove naturally occurring antibodies recognizing mouse RBCs by incubating sera/plasma with wild-type RBCs, centrifuging, and then removing the sera/plasma for further testing. Absorbed sera/plasma was then incubated with either: 1) wild-type RBCs or 2) Jk^{b} transgenic RBCs (as per FIG. 1). After incubation and washing, RBCs were then incubated with a commercially available fluorescently conjugated secondary antibody (anti-IgG) and analyzed by flow cytometry.

Histograms of wild-type and Jk^{b} transgenic RBCs were superimposable for the negative control plasma (FIG. 9A). In sharp contrast, Jk^{b} transgenic RBCs showed a positive shift compared to wild-type RBCs for the plasma from the patient with a known anti-Jk^{b} antibody (FIG. 9B) (confirmed by current FDA approved assay systems). Together, these data demonstrate that this approach works to identify an alloantibody against a well-defined and isolated human RBC antigen. If human RBCs had been used as targets to characterize the antibody from Sample 2, one could not isolate Jk^{b} as the antigen being recognized without using a whole panel of different human RBCs with different antigen combinations.

### Example 2: Detection of Alloantibodies in the Presence of Autoantibodies

This Example describes a representative method for analyzing blood samples for the presence of alloantibodies where the subject from whom the blood sample is derived also expresses one or more autoantibodies. This provides an exemplary method for screening blood as would be beneficial to evaluate blood for treatment (*e.g.,* transfusion) of subjects with autoimmune hemolytic anemia and other conditions that involve autoantibody and alloantibody reactivity.

Existing technologies that use human RBCs cannot detect alloantibodies underlying an autoantibody, as the autoantibody reacts with all RBCs, thus obfuscating detection of the alloantibodies. The best that can be done with previous technology is an "allogeneic" adsorption that can remove the autoantibody - however, in doing so, one risks also removing the alloantibody, and as such, getting a false negative test.

In the technology provided herein, specific alloantigens are expressed on non-human RBCs (exemplified here with mouse RBCs), which do not express human autoantigens. Even if some autoantigens are expressed, having matched pairs of wild-type RBCs and transgenic RBCs allow a clean adsorption of autoantibodies without removing the underlying alloantibody. Thus, using the technology provided herein, one can cleanly detect alloantibodies in a sample, and if present, determine their specificity.

A human blood sample (from which red blood cells were substantially removed) from a hemolytic anemia patient known to have an autoantibody (specifically, a warm antibody) and also an alloantibody against K1 was tested using mouse RBCs expressing the K1 alloantigen, generated as described herein. Wild-type mouse RBCs, not expressing K1, were used as a negative control. Likewise, patient serum without anti-K1 alloantibody was used as a second negative control. Samples were adsorbed using wild-type RBCs, and then confirmed to be nonreactive to the wild-type RBCs. Adsorbed samples were then tested with transgenic RBCs expressing the K1 alloantigen. Antibody binding was determined by flow cytometry, which in previous studies, has given the same results as other available platforms (*e.g.* solid phase, tube testing, gel card, etc.).

Plasma from a hemolytic anemia patient who had both a warm autoantibody and an anti-K1 alloantibody were adsorbed with wildtype RBCs and then tested with transgenic RBCs expressing isolated alloantigens (either K1, K2, or Jk^{b}). There was no reactivity with wild-type, K2, or Jk^{b} RBCs. In contrast, transgenic K1 RBCs showed a strong reactivity. These data demonstrate that the transgenic RBC screening technology described herein can be used to identify alloantibodies in the presence of autoantibodies without the autoantibodies interfering with the assay. This is unique to the antibody detection approach described and cannot be achieved using natural human RBCs

As will be understood by one of ordinary skill in the art, each embodiment disclosed herein can comprise, consist essentially of or consist of its particular stated element, step, ingredient or component. Thus, the terms "include" or "including" should be interpreted to recite: "comprise, consist of, or consist essentially of." The transition term "comprise" or "comprises" means includes, but is not limited to, and allows for the inclusion of unspecified elements, steps, ingredients, or components, even in major amounts. The transitional phrase "consisting of" excludes any element, step, ingredient or component not specified. The transition phrase "consisting essentially of" limits the scope of the embodiment to the specified elements, steps, ingredients or components and to those that do not materially affect the embodiment. A material effect would cause a statistically significant reduction in identification of antibody(s) using one of the methods provided herein.

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques. When further clarity is required, the term "about" has the meaning reasonably ascribed to it by a person skilled in the art when used in conjunction with a stated numerical value or range, *i.e.* denoting somewhat more or somewhat less than the stated value or range, to within a range of ±20% of the stated value; ±19% of the stated value; ±18% of the stated value; ±17% of the stated value; ±16% of the stated value; ±15% of the stated value; ±14% of the stated value; ±13% of the stated value; ±12% of the stated value; ±11% of the stated value; ±10% of the stated value; ±9% of the stated value; ±8% of the stated value; ±7% of the stated value; ±6% of the stated value; ±5% of the stated value; ±4% of the stated value; ±3% of the stated value; ±2% of the stated value; or ±1% of the stated value.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (*e.g.,* "such as") provided herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain embodiments of this invention are described herein, including the best mode known to the inventors for carrying out the invention. Of course, variations on these described embodiments will become apparent to those of ordinary skill in the art upon reading the foregoing description. The inventor expects skilled artisans to employ such variations as appropriate, and the inventors intend for the invention to be practiced otherwise than specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

Furthermore, numerous references have been made to patents, printed publications, journal articles and other written text throughout this specification (referenced materials herein).

In closing, it is to be understood that the embodiments of the invention disclosed herein are illustrative of the principles of the present invention. Other modifications that may be employed are within the scope of the invention. Thus, by way of example, but not of limitation, alternative configurations of the present invention may be utilized in accordance with the teachings herein. Accordingly, the present invention is not limited to that precisely as shown and described.

The particulars shown herein are by way of example and for purposes of illustrative discussion of the preferred embodiments of the present invention only and are presented in the cause of providing what is believed to be the most useful and readily understood description of the principles and conceptual aspects of various embodiments of the invention. In this regard, no attempt is made to show structural details of the invention in more detail than is necessary for the fundamental understanding of the invention, the description taken with the drawings and/or examples making apparent to those skilled in the art how the several forms of the invention may be embodied in practice.

Definitions and explanations used in the present disclosure are meant and intended to be controlling in any future construction unless clearly and unambiguously modified in the following examples or when application of the meaning renders any construction meaningless or essentially meaningless. In cases where the construction of the term would render it meaningless or essentially meaningless, the definition should be taken from Webster's Dictionary, 3rd Edition or a dictionary known to those of ordinary skill in the art, such as the Oxford Dictionary of Biochemistry and Molecular Biology (Eds. Attwood T et al., Oxford University Press, Oxford, 2006).

## Claims

1. A method, comprising:
contacting a control red blood cell (RBC) with a sample;
removing the control RBC to generate a pre-absorbed sample;
contacting at least one transgenic RBC with the pre-absorbed sample; and
determining whether an antibody binds to an antigen;
wherein the at least one transgenic RBC, has been provided from, or derived from, a non-target vertebrate animal, that expresses the antigen from a target species other than the non-target vertebrate animal;
wherein the sample is known or suspected to include the antibody that binds to the antigen and wherein the sample has been provided from the target species;
wherein the control RBC has been provided from the non-target vertebrate animal of the same species that does not express the antigen; and
wherein the target species is human and the non-target vertebrate animal is a non-human mammal.

2. The method of any one of the preceding claims, wherein the antigen comprises an alloantigen.

3. The method of claim 2, wherein:
i. the alloantigen is selected from a blood group system consisting of: ABO, FY, KEL, JK, MNS, GLOB, and Rh blood group systems; or
ii. the alloantigen is selected from the group consisting of: A, B, O, Fya, Fyb, KEL1, KEL2, KPb, KPa, Jsb, Jsa, Jka, Jkb, M, N, S, U, P, RhD, RhCE, Rhce, RhCe, RhcE, f, and G.

4. The method of claim 2, wherein the alloantigen comprises a platelet antigen.

5. The method of claim 4, wherein the platelet antigen is HPA-2a, HPA-2b, HPA-3a, HPA-3b, HPA-4a, HPA-4b, HPA-5a, HPA-5b, HPA-6bw, HPA-7bw, HPA-8bw, HPA-9bw, HPA-10bw, HPA-11bw, HPA-16bw, HPA-17bw, HPA-18bw, HPA-19bw, HPA-20bw, HPA-21bw, HPA-22bw, HPA-23bw, HPA-24bw, HPA-25bw, HPA-26bw, HPA-27bw, HPA-28bw, or HPA-29bw.

6. The method of any one of the preceding claims, wherein:
i. the antigen comprises an autoantigen; or
ii. the antigen comprises a microbial, fungal, viral, or bacterial antigen; or
iii. the antigen comprises a fetal antigen; or
iv. the transgenic RBC expresses two or more distinct antigens from the target species; or
v. the transgenic RBC is isolated from a transgenic non-target vertebrate animal engineered to express the antigen; or
vi. determining whether the antibody binds to the antigen comprises use of an assay selected from the group consisting of: agglutination by tube assay, gel card, flow cytometry, solid phase platforms, spotted antigen arrays, and ELISA.

7. The method of any one of the preceding claims, wherein the method further comprises removing native proteins expressed by the transgenic RBC that are known or considered to be cross-reactive to one or more antibodies in the sample prior to contacting the transgenic RBC with the pre-absorbed sample.

8. The method of claim 7, wherein the removing comprises one or more of:
treatment with periodic acid to remove carbohydrates;
treatment with at least one glycosidase to remove carbohydrates; and
treatment with at least one protease under conditions that remove cross-reactive antigens but do not remove the antigen from the target species.

9. A composition comprising:
a transgenic red blood cell (RBC), from a non-target vertebrate animal, that expresses an antigen from a target species other than the non-target vertebrate animal;
an antibody bound to the antigen, wherein the antibody is from a sample from the target species that has been pre-cleared of unwanted antibodies using a non-transgenic RBC preparation from the non-target vertebrate animal; and
wherein the target species is human and the non-target vertebrate animal is a non-human mammal.

10. The composition of claim 9, wherein the antigen is HPA-2a, HPA-2b, HPA-3a, HPA-3b, HPA-4a, HPA-4b, HPA-5a, HPA-5b, HPA-6bw, HPA-7bw, HPA-8bw, HPA-9bw, HPA-10bw, HPA-11bw, HPA-16bw, HPA-17bw, HPA-18bw, HPA-19bw, HPA-20bw, HPA-21bw, HPA-22bw, HPA-23bw, HPA-24bw, HPA-25bw, HPA-26bw, HPA-27bw, HPA-28bw, or HPA-29bw.

11. The composition of claim 9 or 10, wherein the transgenic RBC expresses two or more distinct antigens from the target species.

12. The composition of any one of claims 9-11, wherein the transgenic RBC has been treated to remove native proteins expressed by the transgenic RBC that are known or considered to be cross-reactive to one or more antibodies from the species other than the non-target mammal.

13. The composition of claim 12, wherein the treatment comprises one or more of:
treatment with periodic acid to remove carbohydrates;
treatment with glycosidases to remove carbohydrates; and
treatment with proteases under conditions that remove cross-reactive antigens but do not remove the antigen from the target species.

## Patentansprüche

1. Verfahren, umfassend:
Inkontaktbringen eines roten Kontroll-Blutkörperchens (Kontroll-RBK) mit einer Probe;
Entfernen des Kontroll-RBK, um eine vorabsorbierte Probe zu erzeugen;
Inkontaktbringen mindestens eines transgenen RBK mit der vorabsorbierten Probe und
Bestimmen, ob ein Antikörper an ein Antigen bindet;
wobei das mindestens eine transgene RBK von einem Nichtziel-Wirbeltier bereitgestellt oder abgeleitet wird, das das Antigen von einer Zielspezies, bei der es sich nicht um das Nichtziel-Wirbeltier handelt, exprimiert;
wobei von der Probe bekannt ist oder angenommen wird, dass sie den Antikörper beinhaltet, der an das Antigen bindet, und wobei die Probe von der Zielspezies bereitgestellt wurde;
wobei das Kontroll-RBK von dem Nichtziel-Wirbeltier derselben Spezies bereitgestellt wurde, das das Antigen nicht exprimiert; und
wobei die Zielspezies menschlich ist und das Nichtziel-Wirbeltier ein nichtmenschliches Säugetier ist.

2. Verfahren nach einem der vorhergehenden Ansprüche, wobei das Antigen ein Alloantigen umfasst.

3. Verfahren nach Anspruch 2, wobei:
i. das Alloantigen ausgewählt ist aus einem Blutgruppensystem bestehend aus: ABO-, FY-, KEL-, JK-, MNS-, GLOB- und Rh-Blutgruppensystemen; oder
ii. das Alloantigen ausgewählt ist aus der Gruppe bestehend aus: A, B, 0, Fya, Fyb, KEL 1, KEL2, KPb, KPa, Jsb, Jsa, Jka, Jkb, M, N, S, U, P, RhD, RhCE, Rhce, RhCe, RhcE, f und G.

4. Verfahren nach Anspruch 2, wobei das Alloantigen ein Plättchenantigen umfasst.

5. Verfahren nach Anspruch 4, wobei das Plättchenantigen HPA-2a, HPA-2b, HPA-3a, HPA-3b, HPA-4a, HPA-4b, HPA-5a, HPA-5b, HPA-6bw, HPA-7bw, HPA-8bw, HPA-9bw, HPA-10bw, HPA-11bw, HPA-16bw, HPA-17bw, HPA-18bw, HPA-19bw, HPA-20bw, HPA-21bw, HPA-22bw, HPA-23bw, HPA-24bw, HPA-25bw, HPA-26bw, HPA-27bw, HPA-28bw oder HPA-29bw ist.

6. Verfahren nach einem der vorhergehenden Ansprüche, wobei:
i. das Antigen ein Autoantigen umfasst; oder
ii. das Antigen eine mikrobielles, Pilz-, Virus- oder bakterielles Antigen umfasst; oder
iii. das Antigen ein fötales Antigen umfasst; oder
iv. das transgene RBK zwei oder mehr verschiedene Antigene von der Zielspezies exprimiert; oder
v. das transgene RBK aus einem transgenen Nichtziel-Wirbeltier isoliert ist, das dazu konstruiert ist, das Antigen zu exprimieren; oder
vi. das Bestimmen, ob der Antikörper an das Antigen bindet, eine Verwendung eines Assays umfasst, der ausgewählt ist aus der Gruppe bestehend aus: Agglutinationby-Tube-Assay, Gelkarte, Durchflusszytometrie, Festphasenplattformen, Spotted-Antigen-Arrays und ELISA.

7. Verfahren nach einem der vorhergehenden Ansprüche, wobei das Verfahren weiterhin ein Entfernen von nativen Proteinen, die von dem transgenen RBK exprimiert werden und von denen bekannt ist oder erwogen wird, das sie zu einem oder mehreren Antikörpern in der Probe kreuz reaktiv sind, vor dem Inkontaktbringen des transgenen RBK mit der vorabsorbierten Probe umfasst.

8. Verfahren nach Anspruch 7, wobei das Entfernen eine oder mehrere der folgenden umfasst:
Behandlung mit Periodsäure, um Kohlenhydrate zu entfernen;
Behandlung mit mindestens einer Glykosidase, um Kohlenhydrate zu entfernen; und
Behandlung mit mindestens einer Protease unter Bedingungen, die kreuzreaktive Antigene entfernen, jedoch nicht das Antigen von der Zielspezies entfernen.

9. Zusammensetzung, umfassend:
ein transgenes rotes Blutkörperchen (RBK) von einem Nichtziel-Wirbeltier, das ein Antigen von einer Zielspezies, bei der es sich nicht um das Nichtziel-Wirbeltier handelt, exprimiert;
einen Antikörper, der an das Antigen gebunden ist, wobei der Antikörper aus einer Probe von der Zielspezies ist, die unter Verwendung eines nichttransgenen RBK-Präparats von dem Nichtziel-Wirbeltier zuvor von unerwünschten Antikörpern gereinigt wurde; und
wobei die Zielspezies menschlich ist und das Nichtziel-Wirbeltier ein nichtmenschliches Säugetier ist.

10. Zusammensetzung nach Anspruch 9, wobei das Antigen HPA-2a, HPA-2b, HPA-3a, HPA-3b, HPA-4a, HPA-4b, HPA-5a, HPA-5b, HPA-6bw, HPA-7bw, HPA-8bw, HPA-9bw, HPA-10bw, HPA-11bw, HPA-16bw, HPA-17bw, HPA-18bw, HPA-19bw, HPA-20bw, HPA-21bw, HPA-22bw, HPA-23bw, HPA-24bw, HPA-25bw, HPA-26bw, HPA-27bw, HPA-28bw oder HPA-29bw ist.

11. Zusammensetzung nach Anspruch 9 oder 10, wobei das transgene RBK zwei oder mehr verschiedene Antigene von der Zielspezies exprimiert.

12. Zusammensetzung nach einem der Ansprüche 9-11, wobei das transgene RBK behandelt wurde, um native Proteine zu entfernen, die von dem transgenen RBK exprimiert werden und von denen bekannt ist oder erwogen wird, das sie zu einem oder mehreren Antikörpern von der Spezies, bei der es sich nicht um das Nichtziel-Säugetier handelt, kreuz reaktiv sind.

13. Zusammensetzung nach Anspruch 12, wobei die Behandlung eine oder mehrere der folgenden umfasst:
Behandlung mit Periodsäure, um Kohlenhydrate zu entfernen;
Behandlung mit Glykosidasen, um Kohlenhydrate zu entfernen; und
Behandlung mit Proteasen unter Bedingungen, die kreuzreaktive Antigene entfernen, jedoch nicht das Antigen von der Zielspezies entfernen.

## Revendications

1. Procédé, comprenant :
la mise en contact d'un globule rouge (GR) témoin avec un échantillon ;
l'élimination du GR témoin pour produire un échantillon préabsorbé ;
la mise en contact d'au moins un GR transgénique avec l'échantillon préabsorbé ; et
le fait de déterminer si un anticorps se lie à un antigène ;
dans lequel l'au moins un GR transgénique a été produit à partir d'un, ou dérivé d'un, animal vertébré non-cible qui exprime l'antigène provenant d'une espèce cible autre que l'animal vertébré non-cible ;
dans lequel l'échantillon est connu pour contenir ou suspecté de contenir l'anticorps qui se lie à l'antigène et dans lequel l'échantillon a été produit à partir de l'espèce cible ;
dans lequel le GR témoin a été produit à partir de l'animal vertébré non-cible de la même espèce qui n'exprime pas l'antigène ; et
dans lequel l'espèce cible est humaine et l'animal vertébré non-cible est un mammifère non humain.

2. Procédé selon l'une quelconque des revendications précédentes, dans lequel l'antigène comprend un alloantigène.

3. Procédé selon la revendication 2, dans lequel :
i. l'alloantigène est sélectionné à partir d'un système de groupes sanguins constitué de : systèmes de groupes sanguins ABO, FY, KEL, JK, MNS, GLOB et Rh ; ou
ii. l'alloantigène est sélectionné dans le groupe constitué de : A, B, 0, Fya, Fyb, KEL1, KEL2, KPb, KPa, Jsb, Jsa, Jka, Jkb, M, N, S, U, P, RhD, RhCE, Rhce, RhCe, RhcE, f, et G.

4. Procédé selon la revendication 2, dans lequel l'alloantigène comprend un antigène plaquettaire.

5. Procédé selon la revendication 4, dans lequel l'antigène plaquettaire est HPA-2a, HPA-2b, HPA-3a, HPA-3b, HPA-4a, HPA-4b, HPA-5a, HPA-5b, HPA-6bw, HPA-7bw, HPA-8bw, HPA-9bw, HPA-10bw, HPA-11bw, HPA-16bw, HPA-17bw, HPA-18bw, HPA-19bw, HPA-20bw, HPA-21bw, HPA-22bw, HPA-23bw, HPA-24bw, HPA-25bw, HPA-26bw, HPA-27bw, HPA-28bw, ou HPA-29bw.

6. Procédé selon l'une quelconque des revendications précédentes, dans lequel :
i. l'antigène comprend un autoantigène ; ou
ii. l'antigène comprend un antigène microbien, fongique, viral ou bactérien ; ou
iii. l'antigène comprend un antigène fœtal ; ou
iv. le GR transgénique exprime deux antigènes distincts ou plus provenant de l'espèce cible ; ou
v. le GR transgénique est isolé à partir d'un animal vertébré transgénique non-cible modifié pour exprimer l'antigène ; ou
vi. le fait de déterminer si l'anticorps se lie à l'antigène comprend l'utilisation d'une analyse sélectionnée dans le groupe constitué de : l'épreuve d'agglutination en éprouvette, la carte de gel, la cytométrie en flux, les plateformes en phase solide, les puces à antigènes et ELISA.

7. Procédé selon l'une quelconque des revendications précédentes, le procédé comprenant en outre l'élimination de protéines natives exprimées par le GR transgénique qui sont connues pour avoir ou considérées comme ayant une réactivité croisée avec un ou plusieurs anticorps présents dans l'échantillon avant la mise en contact du GR transgénique avec l'échantillon préabsorbé.

8. Procédé selon la revendication 7, dans lequel l'élimination comprend un ou plusieurs de :
un traitement avec de l'acide périodique pour éliminer les glucides ;
un traitement avec au moins une glycosidase pour éliminer les glucides ; et
un traitement avec au moins une protéase dans des conditions qui éliminent les antigènes ayant une réactivité croisée, mais n'éliminent pas l'antigène provenant de l'espèce cible.

9. Composition comprenant :
un globule rouge (GR) transgénique, provenant d'un animal vertébré non-cible, qui exprime un antigène provenant d'une espèce cible autre que l'animal vertébré non-cible ;
un anticorps lié à l'antigène, l'anticorps provenant d'un échantillon provenant de l'espèce cible duquel les anticorps indésirables ont été éliminés au préalable en utilisant une préparation de GR non transgéniques provenant de l'animal vertébré non-cible ; et
dans laquelle l'espèce cible est humaine et l'animal vertébré non-cible est un mammifère non humain.

10. Composition selon la revendication 9, dans laquelle l'antigène est HPA-2a, HPA-2b, HPA-3a, HPA-3b, HPA-4a, HPA-4b, HPA-5a, HPA-5b, HPA-6bw, HPA-7bw, HPA-8bw, HPA-9bw, HPA-10bw, HPA-11bw, HPA-16bw, HPA-17bw, HPA-18bw, HPA-19bw, HPA-20bw, HPA-21bw, HPA-22bw, HPA-23bw, HPA-24bw, HPA-25bw, HPA-26bw, HPA-27bw, HPA-28bw, ou HPA-29bw.

11. Composition selon la revendication 9 ou 10, dans laquelle le GR transgénique exprime deux antigènes distincts ou plus provenant de l'espèce cible.

12. Composition selon l'une quelconque des revendications 9 à 11, dans laquelle le GR transgénique a été traité pour éliminer des protéines natives exprimées par le GR transgénique qui sont connues pour avoir ou considérées comme ayant une réactivité croisée avec un ou plusieurs anticorps provenant de l'espèce autre que le mammifère non-cible.

13. Composition selon la revendication 12, dans laquelle le traitement comprend un ou plusieurs de :
un traitement avec de l'acide périodique pour éliminer les glucides ;
un traitement avec des glycosidases pour éliminer les glucides ; et
un traitement avec des protéases dans des conditions qui éliminent les antigènes ayant une réactivité croisée, mais n'éliminent pas l'antigène provenant de l'espèce cible.
